# EUROPEAN PATENT SPECIFICATION

(11) **EP 4 043 542 B1**
(45) Date of publication and mention of the grant of the patent: **31.12.2025**
(21) Application number: 20873974.8
(22) Date of filing: 02.10.2020
(51) Int. Cl.: G16B 20/20, G16H 50/30, C12M 1/00, C12Q 1/6869

(54) **ANALYSIS DEVICE, ANALYSIS METHOD, AND PROGRAM**
ANALYSEVORRICHTUNG, ANALYSEVERFAHREN SOWIE ANALYSEPROGRAMM
PROGRAMME, DISPOSITIF ET PROCÉDÉ D'ANALYSE

(30) Priority: 08.10.2019 JP 2019185444
(43) Date of publication of application: 17.08.2022
(73) Proprietor: The University of Tokyo, Bunkyo-ku, Tokyo 113-8654 (JP); Liquid Mine, Inc., Tokyo 108-0071 (JP)
(72) Inventor: YOKOYAMA Kazuaki, Tokyo 113-8654 (JP); SHIMIZU Eigo, Tokyo 113-8654 (JP); KONDO Kanya, Tokyo 108-0071 (JP)
(74) Representative: Plougmann Vingtoft a/s
(86) International application number: PCT/JP2020/037499
(87) International publication number: WO 2021/070739

(56) References cited:
- WO-A1-2014/175427
- WO-A1-2015/025866
- JP-A- 2015 501 974
- JP-A- 2017 504 846
- JP-A- 2018 513 508
- JP-A- 2018 527 647
- JP-A- 2018 527 661
- JP-A- 2018 537 980
- JP-A- 2019 511 070
- JP-A- H10 502 539
- JP-A- H11 506 609

## Description

### TECHNICAL FIELD

The present invention relates to analysis apparatuses, analysis methods, and programs.

It is widely known that mutations in base sequences contained in the genetic information of somatic cells may cause diseases. Nowadays, information on various somatic mutations, such as what diseases they are associated with, has been collected and recorded in databases and widely used (Non-Patent Document 1).

In addition, due to recent advances in exhaustive base sequence analysis techniques (such as next-generation sequencers), the number of mutations detected in a single mutation analysis has become enormous, ranging from several hundreds to several millions per sample, and it is neither efficient nor practical to interpret the results artificially for each mutation. Accordingly, there is a need for apparatuses that assist humans in the interpretation of analysis results.
Patent Document 2 discloses systems and methods for generating a priority score for a variant of a gene based on its potential significance to a disease. Priority scores may be calculated for multiple variants, and the variants may be ranked based on the generated priority scores.
Patent Document 3 discloses methods and systems for determining the clinical significance of a genetic variant. The methods entail determining, for the variant, (a) a function score based on known impact of the variant on a biological function of a cell or protein, (b) a frequency score based on the frequency of the variant in a population, (c) a co-occurrence score based on how the variant co-occurs with a reference variant having known clinical significance relating to a clinical disease or condition, and (d) a family segregation score based on how the variant segregates with a disease or condition in a family; and aggregating, on a computer, the function score, the frequency score, the co-occurrence score, the family segregation score to generate a clinical significance score indicating the clinical significance of the genetic variant.
Patent Document 4 discloses that increased sensitivity and specificity of characterizing patient-specific variations as mutations that are indicative of a cancer or other disease by identifying patient-specific tumor mutations by comparing tumor and normal sequence reads from the patient and filtering for mutations that are unique to the tumor. By comparing tumor sequence to a normal sequence from the same patient, false-positive mutation calls are minimized in the analysis.

### PRIOR ART DOCUMENT

### NON-PATENT DOCUMENT

NON-PATENT DOCUMENT 1: inet: COSMIC v90, [online], September 5, 2019, [searched on September 30, 2019], Internet <URL: https://cancer.sanger.ac.uk/cosmic>
PATENT DOCUMENT 2: JP 2018 527661 A
PATENT DOCUMENT 3: JP 2017 504846 A
PATENT DOCUMENT 4: JP 2018 513508 A

### SUMMARY OF DISCLOSURE

### PROBLEMS TO BE SOLVED BY THE DISCLOSURE

It is possible to judge whether or not a mutation recorded in the above-described conventional databases has occurred in the sample by analyzing mutations in base sequences utilizing the above-described databases; however, it is impossible to conclude that such mutation is a mutation that directly affects the formation or progression of diseases, such as cancer, in the registered cases (such as a driver mutation for cancer) solely based on the reason that such mutation has occurred. The reason for this is that a wide variety of matters apart from the above should be considered when interpreting the analysis results of mutations. However, no analysis has conventionally been made as to the degree of possibility of being pathologic by combining such a wide variety of items.

The present invention has been made in view of the above circumstances, and an object thereof is to provide an analysis apparatus, an analyzing method, and a program, capable of presenting the degree of possibility of being a mutation affecting the onset or progression of a disease.

### MEANS FOR SOLVING THE PROBLEMS

An analysis apparatus according to a first aspect of the present invention is defined in claim 1.

An analysis method according to a second aspect of the present invention is defined in claim 8.

A program according to a third aspect of the present invention is defined in claim 9.

### EFFECT OF THE DISCLOSURE

According to the present invention, the degree of possibility of being a mutation affecting the onset or progression of a disease can be presented.

### BRIEF DESCRIPTION OF THE DRAWINGS

FIG. 1 is a block diagram showing a configuration example of an analysis apparatus according to an embodiment of the present invention.
FIG. 2 is a functional block diagram representing an example of the analysis apparatus according to the embodiment of the present invention.
FIG. 3 is an explanatory diagram representing an example of mutant base sequence information input to the analysis apparatus according to the embodiment of the present invention.
FIG. 4 is an explanatory diagram representing an example of output information output by the analysis apparatus according to the embodiment of the present invention.
FIG. 5 is a flowchart representing an operation example of the analysis apparatus according to the embodiment of the present invention.
FIG. 6 is an explanatory diagram representing another example of output information output by the analysis apparatus according to the embodiment of the present invention.
FIG. 7 is a diagram showing a configuration of a control unit of a second embodiment.
FIG. 8 is a flowchart showing a processing procedure by the analysis apparatus for determining whether or not there is suspected pathogenicity.

### DESCRIPTION OF THE EMBODIMENTS

### First Embodiment

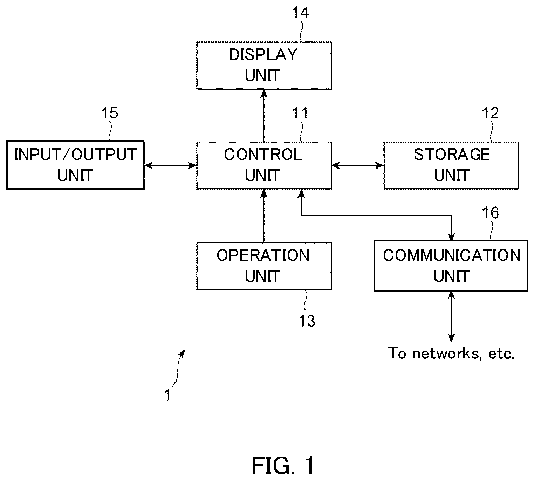

A first embodiment of the present invention will be described with reference to the drawings. As illustrated in FIG. 1, the analysis apparatus 1 according to the embodiment of the present invention is realized with a general computer apparatus including a control unit 11, a storage unit 12, an operation unit 13, a display unit 14, an input/output unit 15, and a communication unit 16.

Here, the control unit 11 is a program control device, such as a CPU. The control unit 11 operates in accordance with a program stored in the storage unit 12. In the present embodiment, the control unit 11 receives, along with sample identification information for identifying an individual to be analyzed and a sample obtained from the individual, mutant base sequence information representing a mutation state, including a mutation location in a base sequence and the content of the mutation, which is extracted, by sequence alignment, from genetic information of the sample. The mutation state may be a mutation of a single base or a structural mutation such as a chromosomal translocation across multiple genes. Specifically, the mutation location and the content of the mutation include: the position where the mutation has occurred (such as information indicating a gene and which base from one side of such gene); and information representing into which base the base that should be present is mutated.

The control unit 11 then sets a score for each mutation state included in the received mutant base sequence information depending on whether or not each of a plurality of predetermined conditions is satisfied for each mutation state represented by the received mutant base sequence information, and further generates synthetic score information in which the set score is synthesized for each mutation state.

Based on the synthetic score information obtained for each mutation state, the control unit 11 estimates a rank representing the degree of possibility of being pathologic for each mutation state, and outputs information on the estimated rank. The operation of the control unit 11 will be described in more detail later.

The storage unit 12 is a memory device, a disk device, or the like. The storage unit 12 retains a program executed by the control unit 11. The storage unit 12 also operates as a work memory of the control unit 11.

The operation unit 13 is a keyboard, a mouse, or the like. The operation unit 13 receives an operation from a user and outputs information representing the operation to the control unit 11. The display unit 14 is a display or the like. The display unit 14 displays information in accordance with an instruction input from the control unit 11.

The input/output unit 15 is, for example, a Universal Serial Bus (USB) interface or the like. The input/output unit 15 performs processing such as receiving information generated by other devices and outputting the information to the control unit 11. The communication unit 16 is a network interface. The communication unit 16 communicates with another computer apparatus connected via a network in accordance with an instruction input from the control unit 11 to transmit and receive data.

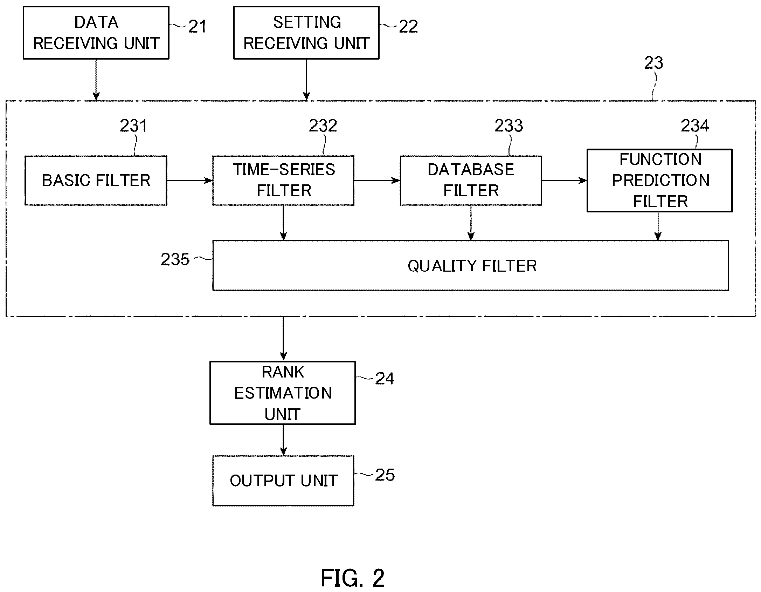

Next, the content of a specific operation of the control unit 11 will be described. In the present embodiment, the control unit 11 functionally realizes the configuration illustrated in FIG. 2 by executing a program stored in the storage unit 12. Namely, the control unit 11 realizes a configuration including a data receiving unit 21, a setting receiving unit 22, a filter processing unit 23, a rank estimation unit 24, and an output unit 25.

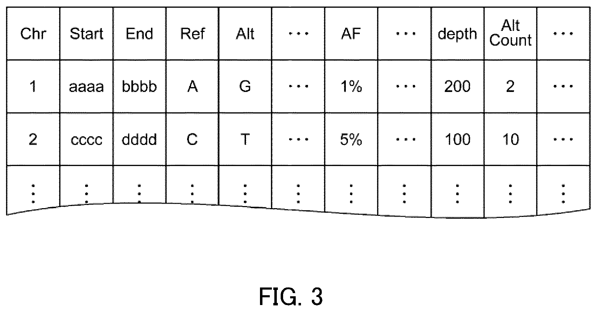

Here, the data receiving unit 21 receives the mutant base sequence information representing the mutation state of the base sequence, which is extracted, by sequence alignment, from the genetic information of the sample to be analyzed. Specifically, as illustrated in FIG. 3, for each mutation state, the number (Chr), a start position (Start), and an end position (End) of the chromosome where a base sequence in such mutation state is found, a base sequence (Ref) that should be originally present, an extracted mutated base sequence (Alt), and a percentage (allele frequency: AF) of the mutated base sequence, are associated with each other in this information. In this example, indices or the like related to quality, such as depth (depth) and count of mutation states (AltCount), are also associated with the above information. It should be noted that the length of the base sequence may be "1" (in this case, the information of the base sequence represents any one of the bases A, T, C, and G).

The mutant base sequence information may also include information related to a case or the like (information such as disease name, treatment history, and tumor percentage) of an individual.

In an example of the present embodiment, the data receiving unit 21 may receive mutant base sequence information (time-series information) extracted from the same individual at a timing (there may be a plurality of timings) different from the timing at which the mutant base sequence information to be analyzed is extracted. In this case, the data receiving unit 21 receives an input designating the mutant base sequence information at the time to be analyzed.

The setting receiving unit 22 receives settings for analysis. This setting is, for example, a setting of which filter(s) to use or a setting of parameter information. A specific example of this setting will be described subsequently, together with the configuration of the filter processing unit 23.

In the present embodiment, the filter processing unit 23 operates to evaluate the possibility of being pathologic based on various kinds of information affecting the interpretation of the analysis result of the mutation. Here, the information affecting the interpretation includes (1) attendant information of the mutation obtained at the time of analysis, and (2) information related to the mutations contained in literature or databases. Among them, the attendant information of the mutation obtained at the time of analysis (1) includes: (a) information on detection accuracy and reliability (the probability that the mutation is not a detection error); (b) allele frequency of the mutation (an index related to the percentage with respect to the whole cell population having the same mutation); (c) time-series information, namely, whether or not the mutation is repeatedly detected in a sample at other time points in the same case; or the like.

The information related to the mutations contained in the literature or databases (2) includes information representing whether or not the mutation is described as a driver mutation of a disease (or how often the description is made). In the event that there is also a registration in the Single Nucleotide Polymorphism (SNP) database, information on the degree of allele frequency of the mutant allele and on whether or not the mutant allele is reported as an SNP in the race may be contained in the literature or databases. Furthermore, information representing whether or not the mutation affects the conformation or function of the encoded protein, for example, whether or not the mutation is shown or predicted by experiment or the like as relating to pathogenesis of cancer, may be contained in the literature or databases as a function prediction.

The filter processing unit 23 judges whether or not a plurality of predetermined conditions are satisfied for each mutation state (in the event that the time-series information is received, the mutation state included in the mutant base sequence information, which is designated as the target of analysis, among the time-series information (hereinafter referred to as a "mutation state to be analyzed")) received by the data receiving unit 21. Specifically, in this embodiment, as illustrated in FIG. 2, the filter processing unit 23 includes a basic filter 231, a time-series filter 232, a database filter 233, a function prediction filter 234, and a quality filter 235.

Here, if the mutation state to be analyzed can be judged to be benign, the basic filter 231 sets a score (e.g., "4") representing that the mutation is benign, and outputs the result to the rank estimation unit 24. If the mutation state to be analyzed cannot be judged to be benign, the basic filter 231 sets a score (e.g., "3") representing that the mutation is not benign, and delivers the processing (passes the processing) to a filter set as the next filter.

Here, the case where the mutation state can be judged to be benign corresponds to: a case where the overlapping portion between the base sequence of a known mutation causing canceration or the like and the mutated base sequence corresponding to the mutation state is relatively short; a case where a region where the mutation represented by the mutation state is located is an intron region; a case where the mutation state is registered in a database in which mutations having no abnormality are accumulated, such as an SNP database; a case where the mutation state can be judged to be benign based on the Gene Damage Index (GDI), or the like.

Here, the GDI is an index representing how much damage is accumulated in a healthy person in terms of each gene, and indicates the possibility that the gene is not considered to come into a pathological condition due to the mutation even if there is a large amount of damage depending on the person (even if there is diversity).

The basic filter 231 receives, from the setting receiving unit 22, at least, any of the settings of: a threshold of the length of an overlapping portion between a base sequence of a known mutation causing canceration or the like and a mutated base sequence corresponding to the mutation state; information specifying a database for judging whether or not the mutation state is an SNP; and a parameter (which is compared with a benignity-judgmental threshold that serves as a criterion for judging whether or not the mutation state is benign or a value registered in a database as a probability of being an SNP) for each database. The basic filter 231 judges whether or not the mutation state to be analyzed is benign based on the received setting.

Specifically, the basic filter 231 sets a score representing that the mutation is benign if the length of the overlapping portion between the base sequence of the known mutation causing canceration or the like and the mutated base sequence corresponding to the mutation state is shorter than a predetermined length threshold. Further, the basic filter 231 sets a score representing that the mutation is benign if the region where the mutation represented by the mutation state is located is an intron region, even if the mutation is not benign when the length of the overlapping portion is shorter than the predetermined length threshold.

Moreover, the basic filter 231 may set a score representing that the mutation is benign based on a result of searching the designated SNP database even if the above-described two conditions are not satisfied. The basic filter 231 sets a score representing that the mutation is benign if, for example, the mutation represented by the mutation state is registered in the SNP database by searching, and a value registered as a probability that the mutation is an SNP exceeds a benignity-judgmental threshold predetermined for the SNP database.

Even if the conditions up to this point are not satisfied, the basic filter 231 refers to the GDI of the gene in which the mutation state is present, and sets a score representing that the mutation is benign if the GDI is larger than a predetermined GDI threshold.

As a result, in the present embodiment, for example, genes having no possibility of becoming driver mutations of cancer (or having a sufficiently low possibility of becoming driver mutations) can be screened out in advance.

In addition, the basic filter 231 may receive, from the setting receiving unit 22, a setting of a condition to be used among the plurality of predetermined conditions for judging benignity (alternatively, a setting of whether or not to pass the processing by setting the score to "3" for all the mutation states without using all the conditions and without operating as the basic filter 231).

In this example, the basic filter 231 makes a judgment as to whether or not the condition(s) is/are satisfied only under the condition(s) set to be used.

If the basic filter 231 passes the processing (the score "3" is set), the time-series filter 232 refers to the information of the mutation state included in the time-series information corresponding to the mutation state to be analyzed, and judges whether or not the same mutation is present in the time-series information extracted at different timings.

The time-series filter 232 uses the mutation state to be analyzed and the corresponding mutation state included in the time-series information, sets, if the same mutations exist, a score (for example, subtracts a first predetermined amount of "1" from the current score) assuming that there is a problematic mutation, and passes the processing to the quality filter 235. The first predetermined amount is, for example, a minimum value subtracted or added in one calculation from the score related to the mutation state. In this example, since the basic filter 231 has passed the processing, the initial score is "3," and when the time-series filter 232 determines that a problematic mutation is present, the first predetermined amount of "1" is subtracted from the score "3" to set the score to "2."

On the other hand, the time-series filter 232 uses the mutation state to be analyzed and the corresponding mutation state included in the time-series information, and sets, if the same mutations do not exist, the score as it is (here, since the initial score is "3," the score is set to "3" without change), and passes the processing to the database filter 233.

It should be noted that the time-series filter 232 may also receive, from the setting receiving unit 22, the setting of a threshold related to the depth, other sequence quality, the mutant allele frequency, and the like. For example, if the depth related to the corresponding mutation state included in the time-series information fails to exceed the threshold set here (e.g., "20"), the time-series filter 232 sets the score as it is (here, since the initial score is "3," the score is set to "3" without change) without judging whether or not the same mutations state are present, and passes the processing to the database filter 233.

Further, in the example of the present embodiment, if the data receiving unit 21 has not received the time-series information (if it has only received the mutant base sequence information to be analyzed as the mutant base sequence information), the time-series filter 232 may set the score as it is (here, since the initial score is "3," the score is set to "3" without change) without judging whether or not the same mutation states are present, and pass the processing to the database filter 233.

If a setting of not using the time-series filter 232 is input from the setting receiving unit 22, the time-series filter 232 sets the score as it is (here, since the initial score is "3," the score is set to "3" without change) without judging whether or not the same mutations states are present, and passes the processing to the database filter 233.

The database filter 233 checks whether or not the mutation state to be analyzed is registered in a predetermined database (e.g., the COSMIC Cancer Database), in which information related to the problematic mutations is accumulated, by transmitting the information related to the mutation state to the server of the database, then, if it is registered, sets a score (e.g., subtracts a first predetermined amount of "1" from the current score) assuming that there is a problematic mutation, and passes the processing to the quality filter 235. In this example, the data base filter 233 makes a judgment on the mutation state to be analyzed when the basic filter 231 has passed the processing and when the time-series filter 232 has passed the processing with the score being kept as it is. Therefore, the database filter 233 subtracts the first predetermined amount of "1" from the thus-obtained score "3" to set the score to "2," and then passes the processing to the quality filter 235.

If the mutation state to be analyzed is not registered in the database in which the information related to the above-described problematic mutation is accumulated, the database filter 233 sets the score as it is, and passes the processing to the function prediction filter 234. In this example, the score at this point remains "3."

Meanwhile, the database filter 233 receives, from the setting receiving unit 22, a setting of a database to be used as the database in which information related to the problematic mutation is accumulated.

This setting may instruct that a plurality of databases should be used, and in this case, the database filter 233 sets a score assuming that there is a problematic mutation if the mutation state to be analyzed is registered in any of the databases in which information related to the problematic mutation is accumulated.

The function prediction filter 234 refers to a database in which the pathogenicity of the mutations is evaluated, sets a score (for example, subtracts a first predetermined amount of "1" from the current score) assuming that there is a pathogenic mutation if the mutation related to the mutation state to be analyzed is registered in the database as having pathogenicity, and passes the processing to the quality filter 235.

Here, as a database in which the pathogenicity of mutations is evaluated, there are widely known databases such as SIFT and PolyPhen2. In addition, some of these databases evaluate the presence or absence of pathogenicity in multiple stages. In such a case, the function prediction filter 234 sets a score (for example, subtracts a first predetermined amount of "1" from the current score) assuming that there is a pathogenic mutation when, for example, in a judgment stage in which the pathogenicity is suspected, and passes the processing to the quality filter 235.

In this example, the function prediction filter 234 make a judgment on the mutation state to be analyzed when the basic filter 231 has passed the processing, when the time-series filter 232 has passed the processing with the score being kept as it is, and when the database filter 233 also has passed the processing with the score being kept as it is. Therefore, the function prediction filter 234 subtracts the first predetermined amount of "1" from the thus-obtained score "3" to set the score to "2," and then passes the processing to the quality filter 235.

Further, the function prediction filter 234 refers to the database in which the pathogenicity of the mutations is evaluated, sets a score as it is if the mutation related to the mutation state to be analyzed is not registered in the database as having pathogenicity (or if it is unclear even when it is registered, or if it is registered as a case of being benign or estimated as being benign), and passes the processing to the quality filter 235. In this example, the score at this point remains "3."

Meanwhile, the function prediction filter 234 also receives, from the setting receiving unit 22, a setting of a database to be used.

The quality filter 235 evaluates the depth of sequencing the mutation state to be analyzed and other qualities of the sequencing of the mutation state to be analyzed. The quality index includes a widely-known index, such as the count number of mutation states, in addition to the depth. The quality filter 235 evaluates the quality by combining the indices (alternatively, by receiving the combination from the setting receiving unit 22 and evaluating in accordance with the received combination of the indices). In the event that a plurality of indices are combined, the quality filter 235 judges that the quality is sufficient if all indices satisfy a condition that the quality is sufficiently high.

The quality filter 235 sets a score (for example, subtracts a first predetermined amount of "1" from the current score) assuming that the judgment is appropriate, and outputs the score to the rank estimation unit 24, when the quality of the sequencing of the mutation state to be analyzed is judged to be sufficient (sufficiently high) by this evaluation. When the quality of the sequencing of the mutation state to be analyzed fails to be judged to be sufficient (sufficiently high), the quality filter 235 sets the score as it is, and outputs the score to the rank estimation unit 24.

One of the features of the present embodiment is that the filter processing unit 23 sets a score based on a judgmental result as to whether or not each condition is satisfied for each mutation state while applying the filters corresponding to a plurality of conditions in a predetermined order.

The rank estimation unit 24 obtains a rank value representing the degree of possibility of being pathologic for each mutation state in accordance with the score for each mutation state output by the filter processing unit 23, and outputs the rank value in association with the information specifying the corresponding mutation state. The rank value representing the possibility of being pathologic may be the value itself of the score output by the filter processing unit 23 corresponding to each mutation state.

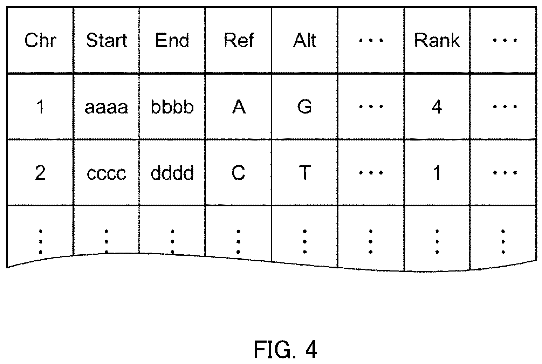

The output unit 25 aggregates and outputs the information specifying each of the mutation states included in the mutant base sequence information and the rank values associated with the respective mutation states, both output by the rank estimation unit 24. The information specifying each of the mutation states in the output information (output information) may be, for example, as illustrated in FIG. 4, the number (Chr), a start position (Start), and an end position (End) of the chromosome where a base sequence in the mutation state included in the mutant base sequence information received by the data receiving unit 21 is found, a base sequence (Ref) that should be originally present, and an extracted mutated base sequence (Alt). In this case, as illustrated in FIG. 4, the output information is arranged in rows in which information specifying each of these mutation states and rank values (Rank) are associated with each other.

### Operation

The present embodiment is configured as described above, and operates basically as follows. The analysis apparatus 1 of the present embodiment reads out and receives mutant base sequence information representing the mutation states in the base sequences extracted, by sequence alignment, from the genetic information of a sample (subject A) to be analyzed as illustrated in FIG. 3 from, for example, a storage device connected to the input/output unit 15.

In this example of the present embodiment, it is assumed that the mutant base sequence information extracted from the subject A, which is the same individual, at a date and time (for example, a date and time before disease treatment) before the date and time (for example, a date and time after the disease treatment) when the mutant base sequence information to be analyzed is extracted, is received as time-series information.

The user of the analysis apparatus 1 performs an operation to set the mutant base sequence information extracted at the date and time after disease treatment as the target of analysis. The analysis apparatus 1 receives this operation to identify the mutant base sequence information to be analyzed and the time-series information.

The user also sets the analysis. In this example of the present embodiment, as the processing for judging a condition(s) for determining a rank, which is the degree of possibility of being pathologic, the following filters are used:
(1) a basic filter for judging a condition by checking, for example, whether or not there is a registration in a database in which benign mutations are registered;
(2) a time-series filter for judging a condition with reference to the time-series information;
(3) a filter (database filter) for judging a condition with reference to a database in which problematic mutations (pathogenic mutations) are registered;
(4) a function prediction filter for judging a condition with reference to a database in which pathogenicity of mutations is evaluated; and
(5) a quality filter for judging a condition by evaluating the quality of sequences.

Here, for the basic filter (1), the setting receiving unit 22 sets a condition to be used among the publicly-known conditions for judging the benignity. Specifically, in the present embodiment, the setting receiving unit 22 sets: a threshold α of the length of the overlapping portion between the base sequence of the known mutation causing the canceration or the like and the mutated base sequence corresponding to the mutation state (if the length is shorter than the threshold α, it is judged to be benign); and a database to be used as the database in which the benign mutations are registered.

For the time-series filter (2), thresholds β and γ relating to, for example, the depth, the mutant allele frequency, and the like, related to the past or the present mutation state (here, thresholds of the depth and the mutant allele frequency) are set. Then, for the database filter (3) and the function prediction filter (4), the setting receiving unit 22 sets a database to be used in each filter. This setting is performed by selecting a database to be used in each filter from options predetermined correspondingly for each filter.

For the quality filter (5), the setting receiving unit 22 selects an index to be used among widely-known indices from predetermined options and sets a threshold of each index. In the example of the following description, the depth and the count number of the mutation states are used, and the depth is set to "20" and the count of the mutation states is set to "10."

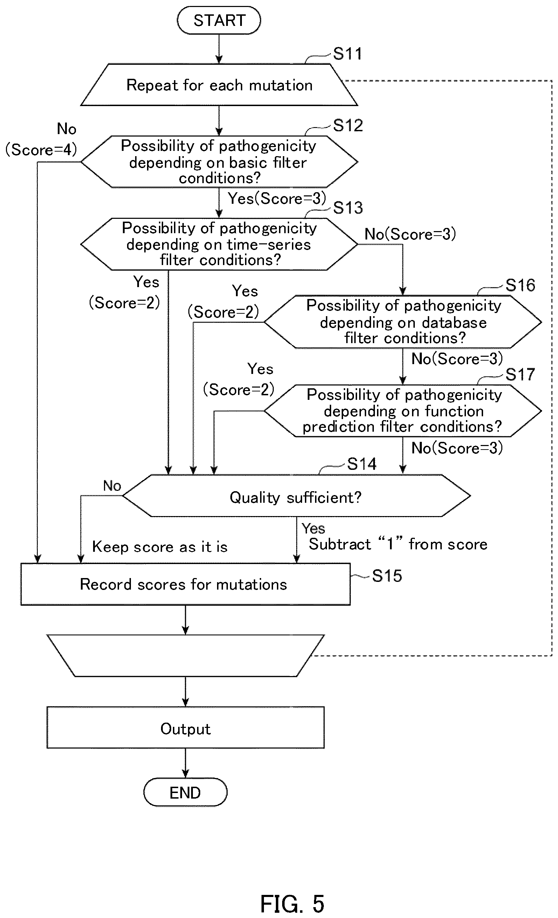

As illustrated in FIG. 5, upon receiving an instruction to start processing from the user, the analysis apparatus 1 sequentially selects each mutation state included in the mutant base sequence information to be analyzed (S11). The analysis apparatus 1 judges whether or not the selected mutation state is suspected of pathogenicity depending on the conditions represented by the basic filter (S12). Specifically, in this example, the threshold α of the length of the overlapping portion between the base sequence of the known mutation causing the canceration or the like and the mutated base sequence corresponding to the mutation state and a database to be used as the database in which the benign mutations are registered, are set. Therefore, in step S12, the analysis apparatus 1 judges whether or not the length of the overlap between the mutation causing the canceration or the like of the selected mutation state and the mutated base sequence corresponding to the mutation state exceeds α and also judges whether or not the selected mutation state is registered in the database in which the benign mutations are registered.

If the length of the overlap between the mutation causing the canceration or the like of the selected mutation state and the mutated base sequence corresponding to the mutation state exceeds α and the selected mutation state is not registered in the database in which the benign mutations are registered, the analysis apparatus 1 judges that pathogenicity is suspected (S12: Yes), and temporarily sets the score related to the selected mutation state to "3," and proceeds to step S13.

Namely, when pathogenicity is suspected in step S12, the analysis apparatus 1 judges whether or not pathogenicity is suspected depending on the conditions represented by the time-series filter (S13). Specifically, the analysis apparatus 1 judges that pathogenicity is suspected (S13: Yes): if the mutation state selected in step S11 is present and the same mutation state corresponding to the selected mutation state is present in the time-series information (the corresponding information is present); if information on the depth related to the corresponding information recorded in the selected mutation state or the time-series information exceeds the predetermined threshold β; and if at least one of the value of the allele frequency of the selected mutation state or the value of the allele frequency related to the corresponding time-series information exceeds the set threshold γ. The analysis apparatus 1 subtracts "1" from the score temporarily set, at this point, as the score related to the mutation state selected, temporarily sets the score related to the selected mutation state to "2," and proceeds to step S14.

When it is judged, in step S13, that pathogenicity is suspected, the analysis apparatus 1 evaluates the quality of the sequencing of the mutation state selected in step S11 depending on the conditions represented by the quality filter (S14).

Specifically, in step S14, the analysis apparatus 1 reads out information related to the quality (in this example, the depth and the mutation state count) recorded in the mutant base sequence information to be analyzed in association with the selected mutation state, and compares with the set thresholds to check whether or not the quality of each piece of information related to the quality is sufficient (in this example, it is judged that the quality is sufficiently high if both exceed the thresholds).

When the analysis apparatus 1 judges that the quality is sufficient (S14: Yes), the analysis apparatus 1 subtracts "1" from the score temporarily set, at this point, as the score related to the mutation state selected to set the score related to the selected mutation state, and records the rank value corresponding to the selected mutation state as the set score in association with the information specifying the selected mutation state (S15).

As a specific example, when the analysis apparatus 1 judges, in step S13, that pathogenicity is suspected, executes step S14 with the score related to the selected mutation state being set to "2," and judges that the quality is sufficient, the score becomes "1" (the rank value is "1").

When the analysis apparatus 1 executes step S14 with the score related to the mutation state being set to "3" by an operation described later and judges that the quality is sufficient, the score becomes "2" (the rank value is "2").

On the other hand, if the analysis apparatus 1 judges that the quality is not sufficient (in this example, either the depth or the mutation state count, both related to quality, fails to exceed a set threshold), the analysis apparatus 1 proceeds to step S15 with the score temporarily set, at this point, as the score related to the selected mutation state being kept as it is, and records the score, as a rank value, in association with the information specifying the selected mutation state.

As a specific example, when the analysis apparatus 1 judges, in step S13, that pathogenicity is suspected, executes step S14 with the score related to the selected mutation state being set to "2," and judges that the quality is not sufficient, the score becomes "2" (the rank value is "2").

When the analysis apparatus 1 executes step S14 with the score related to the mutation state being set to "3" by an operation described later and judges that the quality is not sufficient, the score becomes "3" (the rank value is "3").

Further, in step S13, if the information on the depth related to the selected mutation state or the corresponding information in the time-series information falls below the predetermined threshold β, or alternatively, if both the allele frequency value of the selected mutation state and the allele frequency value related to the corresponding time-series information fall below the set threshold γ, the analysis apparatus 1 judges that no pathogenicity is suspected (S13: No), and proceeds to step S16 with the score temporarily set, at this point, as the score related to the selected mutation state being kept as it is (in this example, "3").

If it is judged, in step S13, that no pathogenicity is suspected, the analysis apparatus 1 judges whether or not the mutation state selected in step S11 is suspected of pathogenicity depending on the conditions represented by the database filter (S16).

Namely, the analysis apparatus 1 accesses a database set as a database to be used via the communication unit 16, and makes an inquiry as to whether or not the mutation state selected in step S11 is registered in the database. If a response is received to the effect that the selected mutation state is registered in the database as a result of the inquiry (S16: Yes), "1" is subtracted from the score "3," at this point, related to the selected mutation state to set the score related to the selected mutation state to "2," and the processing proceeds to and is continued in step S14.

On the other hand, if a response is received to the effect that the selected mutation state is not registered in the database (S16: No) as a result of the inquiry in step S16, the analysis apparatus 1 judges that no pathogenicity is suspected and proceeds to step S17 with the score "3," at this point, related to the selected mutation state being set as it is.

When it is judged, in step S16, that no pathogenicity is suspected, the analysis apparatus 1 judges whether or not the mutation state selected in step S11 is suspected of pathogenicity depending on the condition represented by the function prediction filter (S17).

Namely, the analysis apparatus 1 accesses a database set as a database to be used for the function prediction filter via the communication unit 16, and makes an inquiry as to the degree of pathogenicity of the mutation state selected in step S11 and registered in the database. Here, as a result of the inquiry, when the evaluation of the pathogenicity related to the selected mutation state is high as compared to the predetermined criterion (S17: Yes), the analysis apparatus 1 subtracts "1" from the score "3," at this point, related to the selected mutation state to set the score related to the selected mutation state to "2," and proceeds to step S14 to continue the processing.

If a response is received to the effect that the selected mutation state is not registered in the database, as a result of the inquiry made in step S17, or alternatively, when the evaluation of the pathogenicity related to the selected mutation state is not high as compared to the predetermined criterion (S17: No), the analysis apparatus 1 judges that no pathogenicity is suspected, and proceeds to step S14 with the score "3," at this point, related to the selected mutation state being set as it is.

Further, in step S12, if the length of overlap between the mutation causing canceration or the like in the selected mutation state and the mutated base sequence corresponding to the mutation state does not exceed α or if there is a registration in the database in which the benign mutations are registered, the analysis apparatus 1 judges that there no pathogenicity is suspected (i.e., the mutation is a benign mutation) (S12: No). The analysis apparatus 1 proceeds to step S15 with the score related to the selected mutation state being "4." In this case, the analysis apparatus 1 records the score "4," as a rank value, in association with the information specifying the selected mutation state.

If there is still a mutation state unselected in the mutant base sequence information to be analyzed, the analysis apparatus 1 returns to step S11 to continue the processing by selecting one of the unselected mutation states. On the other hand, if there are no more unselected mutation states (i.e., if rank values are associated with all mutation states included in the mutant base sequence information to be analyzed), output information in which information specifying each mutation state and its rank value are associated with each other is output. This output information is, for example, as illustrated in FIG. 4.

By referring to the output information, the user may judge whether or not the mutation is pathologic by, for example, focusing on the mutations with the rank value of "1" or "2." In addition, in this example, there is a possibility of sequence error if the rank value is "3." Therefore, in the case where there is a large number of mutations with the rank value of "3," the user may judge, for example, that the inspection needs to be performed again.

It should be noted that the flow of operations of the analysis apparatus 1 illustrated here is an example, and the order thereof may be arbitrarily determined by the user. For example, steps S17 and S16 may be reversed. Further, the processing in the steps related to any filter may be omitted.

### Record Related to Judgments

In addition, in the example of the present embodiment, the control unit 11 of the analysis apparatus 1 further generates record information representing which condition and how such condition has been judged (for example, which condition has been satisfied) in the operation (judgment of a plurality of conditions) of a filter executed for each mutation state, among the basic filter 231, the time-series filter 232, the database filter 233, the function prediction filter 234, and the quality filter 235, in the operation of the filter processing unit 23. The control unit 11 may include, in the output information, such record information, along with the rank value for each mutation state, and outputs such output information.

For example, when functioning as the basic filter 231, if the control unit 11 judges that there is a registration in, for example, an SNP database (an example of a database in which mutations without abnormality are accumulated), the control unit 11 sets the score to "4," and outputs record information to the effect that there is a record in the SNP database. When functioning as the output unit 25, the control unit 11 outputs the record information in association with the information specifying the mutation states together with the rank values based on the score values in the output information.

In this case, since the functions as other filters are not executed for the relevant mutation state, the recorded information of the other filters which are not executed is not included.

In addition, when functioning as the basic filter 231, if the control unit 11 fails to make a judgment as to the benignity and passes the processing by setting the score to "3," the control unit 11 may not need to generate the record information for the basic filter 231. Alternatively, the control unit 11 may set the score and output the record information serving as grounds for why the benignity could not be judged, such as no record in the SNP database. In this case, when functioning as the output unit 25, the control unit 11 outputs this record information in association with the information specifying the mutation states together with the rank values based on the score values in the output information.

In the above-described example, since the control unit 11 also executes the function of the time-series filter 232 at this time, the control unit 11 compares the allele frequency of the mutation state to be analyzed with the set threshold to generate and output record information indicating the result.

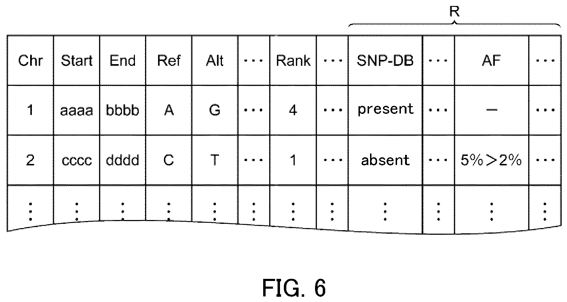

According to such an example, for example, as illustrated in FIG. 6 (corresponding to FIG. 4), the rank values (Rank) and the record information serving as grounds for judgment (in the example of FIG. 6, recorded in columns for each judgmental ground; R) are output in association with information specifying mutation states including: the number (Chr), a start position (Start), and an end position (End) of the chromosome; a base sequence (Ref) to be originally present; and an extracted mutated base sequence (Alt).

When, for example, an evaluation is made that pathogenicity is present (when the rank value is "1"), the user can distinguish and handle whether such judgmental ground is mainly based on the judgment by the time-series filter or based on the judgment by the database filter, or the like, by referring to not only the rank values but also the record information serving as the judgmental grounds. This helps to understand the nature of the mutations.

### Analysis of Recurrence

The analysis apparatus 1 of the first embodiment may also accumulate and record the appearance frequency of each mutation state included in the mutant base sequence information, each time the mutant base sequence information is analyzed (regardless of the sample or individual). In addition, the analysis apparatus 1 may present the recorded appearance frequency of each mutation state to the user. For example, the analysis apparatus 1 may output the cumulative value of the recorded appearance frequency of the mutation state for each mutation state included in the mutant base sequence information to be analyzed.

By referring to the cumulative value information, the user can judge, regarding a mutation state with a rank that is not high (wherein such rank represents the degree of possibility of being pathologic), whether or not such mutation state appears a large number of times regardless of the sample or individual. If such mutation appears a large number of times regardless of the sample or individual, it is possible to make a judgment such as that such mutation is at a site highly prone to read errors by the next-generation sequencer.

Further, by referring to the cumulative value information, the user can judge, regarding a mutation state with a possibility of being pathologic, whether or not such mutation state appears a large number of times regardless of the sample or individual. If such mutation state appears a large number of times regardless of the sample of individual, the user can learn that such mutation state may be a pathological mutation that is unknown heretofore even if such mutation state is not registered in the database.

In addition, the analysis apparatus 1 may further obtain and record the cumulative value of the appearance frequency for each mutation state independent of the sample or individual, for each case information (for example, disease name) included in the mutant base sequence information. Further, a cumulative value of the appearance frequency for each rank value of the mutation state may be obtained and recorded as the cumulative value of the appearance frequency for each mutation state.

Moreover, information on the manufacturer and model name of the next-generation sequencers that performed the sequencing may be included in the mutant base sequence information, and the appearance frequency may be aggregated for each piece of information on the manufacturer and model name of the sequencer. In this case, it is possible to learn the peculiarity of the sequencing error for each manufacturer or model of the sequencer.

As described above, according to the first embodiment, the possibility of being a mutation affecting the onset or progression of a disease, namely, the degree of possibility of being pathologic can be presented, and a contribution can be made to the judgment as to, for example, the degree of possibility of each mutation being a mutation directly affecting the onset or progression of a disease (e.g., a driver mutation for cancer).

### Second Embodiment

The first embodiment has described an example case of presenting the degree of possibility of a single base polymorphism of the sample being pathologic. In contrast, the second embodiment will describe an example case of presenting the degree of possibility of mutations of two or more bases of the sample being pathologic.

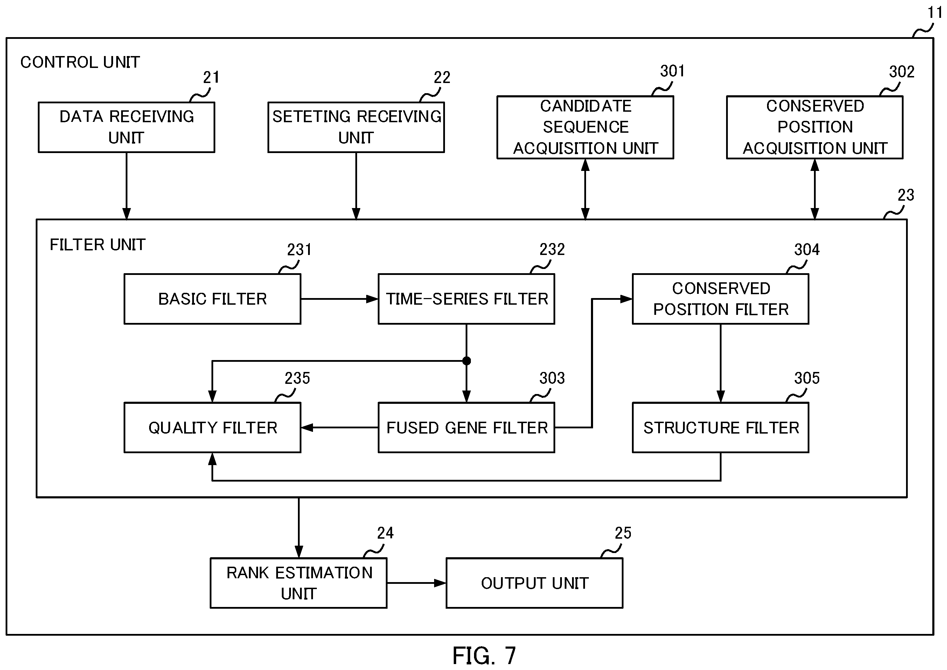

FIG. 7 is a diagram showing the configuration of the control unit 11 according to the second embodiment. The control unit 11 functionally realizes the configuration illustrated in FIG. 7 by executing a program stored in the storage unit 12. The control unit shown in FIG. 7 differs from the configuration of FIG. 2 in that it does not include the database filter 233 and the function prediction filter 234, but includes a candidate sequence acquisition unit 301, a conserved position acquisition unit 302, a fusion gene filter 303, a conserved position filter 304, and a structure filter 305. Functional blocks which are the same as those in FIG. 2 are denoted by the same reference numerals, and the description thereof will be omitted.

It is known that two genes of a specific combination are fused due to translocation, inversion or the like of a chromosome, thereby causing proliferation of cancer cells. For example, a BCR-ABL fusion gene, in which a BCR gene and an ABL gene are fused due to a translocation of a chromosome, is known to proliferate leukemic cells. The storage unit 12 stores, for each fusion gene, a base sequence in which a plurality of combinations of candidate genes is encoded, which is known to cause a driver mutation in a fusion gene in which two candidate genes of a specific combination are fused. For example, a base sequence in which the BCR gene and the ABL gene are encoded is stored in the storage unit 12.

The candidate sequence acquisition unit 301 acquires, for each fusion gene in which candidate genes of a specific combination are fused (hereinafter, referred to as a "first fusion gene), base sequences of two candidate genes serving as driver mutation candidates in the first fusion gene. In the example of the second embodiment, the candidate sequence acquisition unit 301 acquires, for each first fusion gene, from the storage unit 12, the respective base sequences of the two candidate genes included in a plurality of first fusion genes stored in the storage unit 12.

The base sequences, in which the candidate genes of the plurality of first fusion genes are encoded, may also be stored in an external server (not shown). The candidate sequence acquisition unit 301 may acquire, for each first fusion gene, base sequences, in which two candidate genes of the first fusion gene are encoded, from the external server via the communication unit 16.

A fusion gene in which a specific candidate gene and another gene are fused may cause proliferation of cancer cells. For example, a fusion gene in which an ALK gene is fused to another gene is known to cause proliferation of cancer cells. The storage unit 12 stores base sequences of a plurality of candidate genes serving as driver mutation candidates in a fusion gene fused to another gene (hereinafter, also referred to as a "second fusion gene").

The candidate sequence acquisition unit 301 acquires base sequences of candidate genes serving as driver mutation candidates in the second fusion gene fused to another gene. For example, the candidate sequence acquisition unit 301 acquires the base sequences of the candidate genes of a plurality of second fusion genes from the storage unit 12. The candidate sequence acquisition unit 301 may acquire the base sequences of the candidate genes of the plurality of second fusion genes from an external server via the communication unit 16.

The conserved position acquisition unit 302 acquires conserved sequence position information indicating the position of a conserved sequence which is a base sequence conserved between genomes of different organism species. For example, the conserved position acquisition unit 302 acquires the conserved sequence position information from the storage unit 12. The conserved position acquisition unit 302 may acquire the conserved sequence position information from an external server via the communication unit 16.

The basic filter 231 is the same as that in FIG. 2, except that processing specific to a single base polymorphism is not executed. If the mutation state to be analyzed can be judged to be benign, the basic filter 231 sets a score (e.g., "4") representing that the mutation is benign, and outputs the result to the rank estimation unit 24. Further, if the mutation state to be analyzed cannot be judged to be benign, the basic filter 231 sets a score (e.g., "3") representing that the mutation is not benign, and passes the processing to a filter set as the next filter.

The basic filter 231 receives, from the setting receiving unit 22: the information specifying the threshold of the length of the overlapping portion between the base sequence of the known mutation causing canceration or the like and the mutated base sequence corresponding to the mutation state; and the setting of the parameter (which is compared with a value registered as a benignity-judgmental threshold or the like that serves as a criterion for judging whether or not the mutation state is benign) for each database, and judges whether or not the mutation state to be analyzed is benign based on the setting.

Specifically, the basic filter 231 sets a score representing that the mutation is benign if the length of the overlapping portion between the base sequence of the known mutation causing canceration or the like and the mutated base sequence corresponding to the mutation state is shorter than a predetermined length threshold. Further, even if the above is not the case, the basic filter 231 sets a score representing that the mutation is benign if the region where the mutation represented by the mutation state is located is an intron region.

Moreover, even if the above-described two conditions are not satisfied, the basic filter 231 may set a score representing that the mutation is benign if a search is conducted in a designated database and the mutation represented by the mutation state is registered in the database by the search and if a value registered as a probability of the mutation exceeds a benignity-judgmental threshold which is predetermined for the database.

The time-series filter 232 is the same as that of the first embodiment, except that the value to be subtracted from the score corresponding to the mutation state to be analyzed is different from that of the first embodiment, and that the output destination of the score after calculation by the time-series filter 232 is different from that of the first embodiment. The time-series filter 232 refers to the information of the mutation state included in the time-series information corresponding to the mutation state to be analyzed, and judges whether or not the same mutation is present in the time-series information extracted at different timings.

The time-series filter 232 uses the mutation state to be analyzed and the corresponding mutation state included in the time-series information, and, if the same mutations exist, determines a score corresponding to the mutation state to be analyzed (for example, subtracts a second predetermined amount of "2" from the score) assuming that there is a possibility of being pathologic, and passes the processing to the quality filter 235. In this example, since the basic filter 231 has passed the processing, the initial score is "3." Here, when the time-series filter 232 judges that there is a possibility of being pathologic, the second predetermined amount of "2" is subtracted from the score "3" to set the score to "1." The second predetermined amount is a value greater than the first predetermined amount.

On the other hand, the time-series filter 232 uses the mutation state to be analyzed and the corresponding mutation state included in the time-series information, and sets, if the same mutations do not exist, the score as it is (here, since the initial score is "3," the score is set to "3" without change), and passes the processing to the database filter 233.

It should be noted that the time-series filter 232 may also receive, from the setting receiving unit 22, the setting of a threshold related to the depth, other sequence quality, the mutant allele frequency, and the like. For example, if the depth related to the corresponding mutation state included in the time-series information fails to exceed the threshold set here (e.g., "20"), the time-series filter 232 sets the score as it is (here, since the initial score is "3," the score is set to "3" without change) without judging whether or not the same mutations state are present, and passes the processing to the database filter 233.

Further, similarly to the first embodiment, if the data receiving unit 21 has not received the time-series information (if it has only received the mutant base sequence information to be analyzed as the mutant base sequence information), the time-series filter 232 may set the score as it is (here, since the initial score is "3," the score is set to "3" without change) without judging whether or not the same mutation states are present, and pass the processing to the database filter 233.

If a setting of not using the time-series filter 232 is input from the setting receiving unit 22, the time-series filter 232 sets the score as it is (here, since the initial score is "3," the score is set to "3" without change) without judging whether or not the same mutation states are present, and passes the processing to the fusion gene filter 303.

### Determination as to Whether or Not Base Sequence Similar to Fusion Gene is Included

Hereinafter, a mutated base sequence corresponding to any mutation state included in the mutant base sequence information is also referred to as a "mutant base sequence." The fusion gene filter 303 determines whether or not a fusion gene, in which two genes respectively similar to the two candidate genes of the first fusion gene acquired by the candidate sequence acquisition unit 301 are fused, is included in the mutant base sequence. More specifically, the fusion gene filter 303 determines, for each of the plurality of first fusion genes acquired by the candidate sequence acquisition unit 301, whether or not the degree of similarity between: each of the two base sequences in which the two candidate genes of the first fusion gene are encoded; and at least some base sequences included in the mutant base sequence, is equal to or greater than a threshold. The degree of similarity is expressed by, for example, a ratio of alignments of the two base sequences coinciding with each other. If the ratio of alignments of the two base sequences coinciding with each other is equal to or greater than the threshold, the two base sequences are determined to be similar to each other.

As an example, the fusion gene filter 303 determines, in a BCR-ABL first fusion gene, in which a BCR gene and an ABL gene are fused, acquired by the candidate sequence acquisition unit 301, the degree of similarity between the base sequence in which the BCR gene is encoded and the corresponding base sequence in the mutant base sequence. Next, the fusion gene filter 303 determines, in the BCR-ABL first fusion gene, the degree of similarity between the base sequence in which the ABL gene is encoded and the corresponding base sequence in the mutant base sequence.

The fusion gene filter 303 determines whether or not the determined two degrees of similarity are both equal to or greater than a threshold. The threshold is, for example, a value at which the activity of a protein in which the first fusion gene is encoded and the activity of a protein indicated by the mutant base sequence are assumed to be the same.

If the determined two degrees of similarity are both equal to or greater than the threshold, the fusion gene filter 303 determines that a fusion gene, in which two genes respectively similar to the two candidate genes of the first fusion gene are fused, is included in the mutant base sequence.

On the other hand, if at least one of the determined two degrees of similarity is less than the threshold, the fusion gene filter 303 repeats the same determination for another first fusion gene acquired by the candidate sequence acquisition unit 301. If at least one of the determined two degrees of similarity is less than the threshold for all the first fusion genes acquired by the candidate sequence acquisition unit 301, the fusion gene filter 303 determines that a fusion gene in which two genes respectively similar to the two candidate genes of the first fusion gene are fused is not included in the mutant base sequence for any first fusion gene.

In addition, the fusion gene filter 303 may determine that a fusion gene, in which two genes respectively similar to the two candidate genes of the first fusion gene are fused, is included in the mutant base sequence if the degree of similarity between each base sequence of the two candidate genes of the first fusion gene acquired by the candidate sequence acquisition unit 301 and each base sequence of the two genes of the fusion gene included in the mutant base sequence is between 65% and 100%, inclusive. Preferably, the fusion gene filter 303 may determine that a fusion gene, in which two genes respectively similar to the two candidate genes of the first fusion gene are fused, is included in the mutant base sequence if the degree of similarity between each base sequence of the two candidate genes of the first fusion gene and each base sequence of the two genes of the fusion gene included in the mutant base sequence is between 80% and 100%, inclusive.

Further, the fusion gene filter 303 may transmit the mutant base sequence corresponding to the mutation state to be analyzed to an external server in which combinations of candidate genes of the plurality of first fusion genes are stored. The fusion gene filter 303 checks whether or not a fusion gene having two genes respectively similar to the two candidate genes of the first fusion gene registered in a database of the external server is included in the mutant base sequence. The fusion gene filter 303 may determine that a fusion gene, in which two genes respectively similar to the two candidate genes of the first fusion gene are fused, is included in the mutant base sequence upon receipt of a notification, from the external server, indicating that a fusion gene having two genes respectively similar to the two candidate genes of any one of the plurality of first fusion genes registered in the database of the external server is included in the mutant base sequence.

The fusion gene filter 303 determines whether or not a fusion gene, in which a gene having a base sequence similar to the base sequence of the candidate gene of the second fusion gene acquired by the candidate sequence acquisition unit 301 and another gene are fused, is included in the mutant base sequence. More specifically, the fusion gene filter 303 determines, for each of the plurality of second fusion genes acquired by the candidate sequence acquisition unit 301, the degree of similarity between the base sequence of the candidate gene of the second fusion gene and the base sequence of one of the genes of the fusion gene included in the mutant base sequence. The fusion gene filter 303 determines whether or not the determined degree of similarity is equal to or greater than a threshold. The threshold is a value at which the activity of a protein in which the second fusion gene is encoded and the activity of a protein indicated by the mutant base sequence are assumed to be the same.

The fusion gene filter 303 determines that the mutant base sequence includes a fusion gene having a gene similar to the candidate gene of the second fusion gene acquired by the candidate sequence acquisition unit 301 if the determined degree of similarity is equal to or greater than the threshold. If the determined degree of similarity is less than the threshold, the fusion gene filter 303 repeats the same determination for the candidate gene of another second fusion gene acquired by the candidate sequence acquisition unit 301. If the determined degree of similarity is less than the threshold for all the second fusion genes acquired by the candidate sequence acquisition unit 301, the fusion gene filter 303 determines that the mutant base sequence does not include a fusion gene having a gene similar to the candidate gene of any second fusion gene.

Further, the fusion gene filter 303 may determine that a fusion gene, in which a gene having a base sequence similar to the base sequence of the candidate gene of the second fusion gene and another gene are fused, is included in the mutant base sequence if the degree of similarity between the base sequence of the candidate gene of the second fusion gene acquired by the candidate sequence acquisition unit 301 and the base sequence of one of the genes in the fusion gene included in the mutant base sequence is between 65% and 100%, inclusive. Preferably, the fusion gene filter 303 may determine that a fusion gene, in which a gene having a base sequence similar to the base sequence of the candidate gene of the second fusion gene and another gene are fused, is included in the mutant base sequence if the degree of similarity between the base sequence of the candidate gene of the second fusion gene and the base sequence of one of the genes in the fusion gene included in the mutant base sequence is between 80% and 100%, inclusive.

Further, the fusion gene filter 303 may transmit the mutant base sequence to an external server in which a plurality of second fusion genes is stored. The fusion gene filter 303 checks whether or not the mutant base sequence includes a fusion gene having a gene similar to any of the candidate genes of the plurality of second fusion genes registered in a database of the external server. The fusion gene filter 303 may determine that the mutant base sequence includes a gene similar to the candidate gene of the second fusion gene upon receipt of a notification, from the external server, indicating that the mutant base sequence includes the fusion gene having a gene similar to any of the candidate genes of the plurality of registered second fusion genes.

The fusion gene filter 303 determines a score depending on a determination result of whether or not a fusion gene, in which two genes respectively similar to the two candidate genes of the first fusion gene are fused, is included in the mutant base sequence. For example, for any of the plurality of first fusion genes acquired by the candidate sequence acquisition unit 301, if the fusion gene filter 303 determines that a fusion gene, in which two genes respectively similar to the two candidate genes of the first fusion gene are fused, is included in the mutant base sequence, the fusion gene filter 303 determines a score corresponding to the mutation state to be analyzed as having a possibility of being pathologic (for example, subtracts the second predetermined amount of "2" from the score), and passes the processing to the quality filter 235.

In this way, by referring to the base sequences of the two candidate genes of the first fusion gene, which are known to be relatively highly likely to be driver mutations, the fusion gene filter 303 can accurately estimate the degree of possibility that the mutation state is pathologic by means of scores.

The fusion gene filter 303 determines a score depending on a determination result of whether or not the mutant base sequence includes a fusion gene, in which a gene having a base sequence similar to the base sequence of the candidate gene of the second fusion gene and another gene are fused. For example, if the fusion gene filter 303 determines that the mutant base sequence includes a gene similar to the candidate gene of any of the plurality of second fusion genes acquired by the candidate sequence acquisition unit 301, the fusion gene filter 303 determines a score corresponding to the mutation state to be analyzed as having a possibility of being pathologic (for example, subtracts the first predetermined amount of "1" from the score), and passes the processing to the conserved position filter 304.

If the fusion gene filter 303 determines that a fusion gene having candidate genes respectively similar to the two candidate genes of the first fusion gene acquired by the candidate sequence acquisition unit 301 is not included in the mutant base sequence, or if the fusion gene filter 303 determines that a fusion gene having genes similar to the candidate genes of the second fusion gene is not included in the mutant base sequence, the fusion gene filter 303 sets the score as it is (here, since the initial score is "3," the fusion gene filter 303 sets the score to "3" without change), and passes the processing to the conserved position filter 304.

Even if one in a combination of two candidate genes of a fusion gene is not registered in the storage unit 12, it is known that the second fusion gene including a specific candidate gene may be a driver mutation. By referring to the base sequences of the candidate genes of the second fusion gene, the fusion gene filter 303 can accurately present the degree of possibility that the mutation state is pathologic by means of scores.

### Determination as to Whether or Not Position of Conserved Sequence is Included in Mutation Location

Conserved sequences conserved between genomes of different organism species often play an important role in the physiological activity of cells. Therefore, in the event that a mutation occurs at the position of the conserved sequence, the possibility that the mutation state is pathologic is relatively high. The conserved position filter 304 determines a score depending on whether or not the position of a conserved sequence, which is a base sequence conserved between genomes of different organism species, is included in a mutation location in the mutation state. More specifically, the conserved position filter 304 determines whether or not the position of a conserved sequence indicated by the conserved sequence position information acquired by the conserved position acquisition unit 302 is included in the mutation location.

If the conserved position filter 304 determines that the position of the conserved sequence is included in the mutation location, the conserved position filter 304 determines a score corresponding to the mutation state to be analyzed as having a possibility of being pathologic (for example, subtracts the first predetermined amount of "1" from the score), and passes the processing to the structure filter 305. On the other hand, if the conserved position filter 304 determines that the position of the conserved sequence is not included in the mutation location, the conserved position filter 304 sets the score as it is, and passes the processing to the structure filter 305. In this way, by utilizing the information indicating the position of the conserved sequence, the conserved position filter 304 can accurately present the degree of possibility that the mutation state corresponding to the mutation location is pathologic by means of scores.

### Determination of Presence or Absence of Structural Mutation

In the event that structural mutations such as chromosome translocations, deletions of important genes, and mutations across multiple genes occur, it is known that the possibility of these structural mutations being pathologic is relatively high. The structure filter determines whether or not the mutation state represented by the mutant base sequence information is a structural mutation such as a chromosome translocation.

The structure filter 305 determines whether or not the mutation state represented by the mutant base sequence information is a chromosome translocation, and determines a score depending on the determination result. The structure filter 305 refers to the content of the mutation and/or the mutation location included in the mutation states indicated by the mutant base sequence information to determine whether or not the chromosome translocation has occurred. Further, the structure filter 305 may divide the mutant base sequence corresponding to the mutation state into a plurality of base sequences and specify a position on the genome for each of the divided base sequences to thereby determine whether or not the mutation state is a chromosome translocation.

The structure filter 305 determines whether or not the mutation state represented by the mutant base sequence information is a mutation across multiple genes, and determines a score depending on the determination result. The structure filter 305 refers to the content of the mutation and/or the mutation location included in any of the mutation states indicated by the mutant base sequence information to determine whether or not a mutation has occurred across multiple genes. The structure filter 305 may divide the mutant base sequence corresponding to the mutation state into a plurality of base sequences and specify a position on the genome for each of the divided base sequences to thereby determine whether or not the mutation state is a mutation across multiple genes.

Information indicating a plurality of registered genes involved in cell canceration and the like is registered in advance in the storage unit 12. The information indicating the registered genes is, for example, identification information for identifying the registered genes or information indicating the positions of the registered genes on the chromosome. The structure filter 305 may determine whether or not the mutation state represented by the mutant base sequence information is a deletion of a registered gene, and determine a score depending on the determination result. The structure filter 305 refers to the content of the mutation and/or the mutation location included in any of the mutation states indicated by the mutant base sequence information to determine whether or not any of the plurality of registered genes registered in the storage unit 12 has been deleted.

Information of positions on the chromosome of an enhancer that controls expression of genes involved in cell canceration and the like is registered in advance in the storage unit 12. In the event that the structure filter 305 determines that a translocation, a reverse position, a deletion, or the like has occurred, the structure filter 305 may determine whether or not a cancer gene, whose mutation state represented by the mutant base sequence information is registered in the storage unit 12, is a deregulation abnormality located in the vicinity of the enhancer registered in the storage unit 12, and determine the score depending on the determination result.

Information of directions (5'-to-3', 3'-to-5') of gene regions in the genome is registered in advance in the storage unit 12. In the event that the structure filter 305 determines that the mutation state represented by the mutant base sequence information forms a fusion gene, such as a first fusion gene or a second fusion gene, by a translocation, a deletion, or the like, if the two genes forming the fusion gene are assumed to be a first candidate gene and a second candidate gene, the structure filter 305 may determine whether or not the first candidate gene and the second candidate gene are oriented in the same direction (for example, whether there is a combination of the first candidate gene in a 5'-to-3' direction and the second candidate gene also in a 5'-to-3' direction, or alternatively, the first candidate gene in a 3'-to-5' direction and the second candidate gene also in a 3'-to-5' direction), determine whether or not a functional fusion gene is formed, and determine a score depending on the determination result.

Sequence information relating to amino acid translation (codon) of a gene region and RNA splicing is registered in advance in the storage unit 12. In the event that the structure filter 305 determines that the mutation state represented by the mutant base sequence information forms a fusion gene by a translocation, a deletion, or the like, the structure filter 305 may determine whether or not a functional fusion gene is formed based on the information of the above-described items, and determine a score depending on the determination result.

Further, the structure filter 305 divides the mutant base sequence into a plurality of base sequences, and identifies the position on the genome for each of the divided base sequences. The structure filter 305 may compare the position of the specified base sequence on the genome with the positions of a plurality of registered genes registered in the storage unit 12 to determine whether or not a deletion of any registered gene has occurred.

In the event that the structure filter 305 determines that a translocation has occurred, the structure filter 305 determines a score corresponding to the mutation state to be analyzed as having a possibility of being pathologic. For example, the structure filter 305 subtracts the first predetermined amount of "1" from the score corresponding to the mutation state. On the other hand, in the event that it is determined that no translocation has occurred, the score corresponding to the mutation state to be analyzed is kept as it is.

In the event that the structure filter 305 determines that a mutation across multiple genes has occurred, the structure filter 305 determines a score corresponding to the mutation state to be analyzed as having a possibility of being pathologic (for example, subtracts the first predetermined amount of "1" from the score corresponding to the mutation state). On the other hand, in the event that the structure filter 305 determines that no structural mutation has occurred across multiple genes, the score corresponding to the mutation state is kept as it is.

In the event that the structure filter 305 determines that any of the plurality of registered genes registered in the storage unit 12 is deleted, the structure filter 305 further subtracts the first predetermined amount from the score corresponding to the mutation state to be analyzed, and passes the processing to the quality filter 235. On the other hand, in the event that the structure filter 305 determines that none of the plurality of genes registered in the storage unit 12 is deleted, the structure filter 305 keeps the score corresponding to the mutation state to be analyzed as it is, and passes the processing to the quality filter 235. In this way, by determining whether or not structural mutations such as chromosome translocations, mutations across multiple genes, deletion of genes involved in cell canceration, and the like, have occurred, the structure filter 305 can accurately present the degree of possibility that the mutation state is pathologic by means of scores.

### Processing Procedure of Score Evaluation of Mutation States

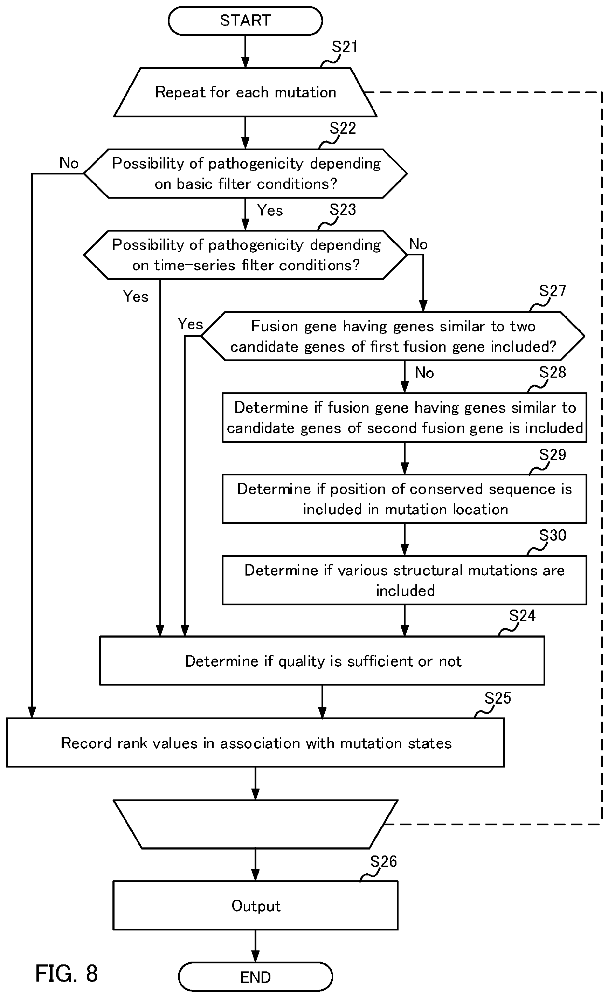

FIG. 8 is a flowchart showing a processing procedure in which the analysis apparatus 1 presents the degree of possibility that a plurality of mutation states included in the mutant base sequence information are pathologic. This processing procedure is started, for example, when an operation receiving unit (not shown) receives a user instruction for starting processing of presenting the degree of possibility that the mutation state is pathologic.

First, the basic filter 231 sequentially selects each mutation state to be analyzed included in the mutant base sequence information (S21), and determines whether or not the selected mutation state has a possibility of pathogenicity depending on the conditions represented by the basic filter, in the same manner as in S12 of FIG. 5 (S22).

Then, if the basic filter 231 determines that the selected mutation state has the possibility of pathogenicity (Yes in S22), the basic filter 231 temporarily sets a score corresponding to the selected mutation state as a score indicating that the mutation is not benign, and proceeds to step S23.

Similarly to S13 of FIG. 5, the time-series filter 232 determines whether or not there is a possibility of pathogenicity depending on the conditions represented by the time-series filter (S23), and sets a score depending on the determination result. If the time-series filter 232 determines that the mutation state has the possibility of pathogenicity (Yes in S23), the time-series filter 232 determines whether or not the quality is sufficient depending on the conditions represented by the quality filter 235 (S24), and determines a score depending on the determination result. The quality filter 235 sets the determined score as a rank value corresponding to the selected mutation state, and records the rank value in association with the information specifying the selected mutation state (S25).

The rank estimation unit 24 repeats the processing from S22 until the rank values are recorded in association with the information specifying all the mutation states included in the mutant base sequence information. The output unit 25 integrates and outputs the information specifying each of the mutation states included in the mutant base sequence information and the rank value associated with each of the mutation states (S26), and ends the processing.

If the time-series filter 232 determines, in step S23, that there is no possibility of pathogenicity (No in S23), the fusion gene filter 303 determines whether or not the mutant base sequence corresponding to the selected mutation state includes a fusion gene in which genes similar to two candidate genes of the first fusion gene are fused (S27). The fusion gene filter 303 determines a score depending on the determination result.

If the fusion gene filter 303 determines that the mutant base sequence does not include the fusion gene in which genes similar to two candidate genes of the first fusion gene are fused (No in S27), the fusion gene filter 303 determines whether or not the mutant base sequence includes a fusion gene having genes similar to candidate genes of the second fusion gene (S28). The fusion gene filter 303 determines a score depending on the determination result. The conserved position filter 304 determines whether or not the position of the conserved sequence is included in the mutation location corresponding to the selected mutation state (S29), and determines a score depending on the determination result.

The structure filter 305 determines whether or not the selected mutation state includes various structural mutations (S30). For example, the structure filter 305 determines whether or not the selected mutation state is a chromosome translocation, and determines a score depending on the determination result. The structure filter 305 determines whether or not the selected mutation state is a mutation across multiple genes, and determines a score depending on the determination result. The structure filter 305 determines whether or not the selected mutation state is a mutation in which any of the plurality of registered genes registered in the storage unit 12 is deleted, and determines a score depending on the determination result. The structure filter 305 executes each determination in S30, and then proceeds to the processing of S24.

If the fusion gene filter 303 determines, in S27, that the mutant base sequence includes a fusion gene in which genes similar to the two candidate genes of the first fusion gene are fused (Yes in S27), the processing proceeds to the determination in S24.

### REFERENCES

- 1: analysis apparatus
- 11: control unit
- 12: storage unit
- 13: operation unit
- 14: display unit
- 15: input/output unit
- 16: communication unit
- 21: data receiving unit
- 22: setting receiving unit
- 23: filter processing unit
- 24: rank estimation unit
- 25: output unit
- 231: basic filter
- 232: time-series filter
- 233: database filter
- 234: functional prediction filter
- 235: quality filter
- 301: candidate sequence acquisition unit
- 302: conserved position acquisition unit
- 303: fusion gene filter
- 304: conserved position filter
- 305: structure

## Claims

1. An analysis apparatus for analyzing a mutation state, including a mutation location in a base sequence and the content of the mutation, extracted from genetic information of a sample to be analyzed by sequence alignment, comprising:
a receiving unit (21) that receives mutant base sequence information representing the mutation state;
a filter processing unit (23) that outputs a score determined depending on whether or not the mutation state represented by the mutant base sequence information received by the receiving unit (21) is a structural mutation across multiple genes;
an output unit (25) that creates rank information representing the degree of possibility that the mutation state is pathologic on the basis of the score output by the filter processing unit (23) and outputs the created rank information;
a retaining unit that retains the mutant base sequence information received by the receiving unit (21), and
a storage unit (12) that stores positions on a chromosome of an enhancer that controls expression of genes involved in cell canceration, wherein
the filter processing unit (23) further includes
a database filter (233) that determines whether or not the mutation state is registered in an external apparatus as a mutation with pathogenicity,
a function prediction filter (234) that evaluates possibility of being a mutation causing a disease by increasing or decreasing the score depending on a prediction result of predicting whether or not the mutation state affects protein function if the database filter (233) determines that the mutation state is not registered in the external apparatus as the mutation with pathogenicity,
a time-series filter (232) that evaluates possibility of being a mutation causing the disease by increasing or decreasing the score depending on a determination result of determining whether or not the mutant base sequence information that is extracted from genetic information of an individual common to the mutant base sequence information currently received by the receiving unit (21) and that is previously received by the receiving unit (21) represents the same mutation state as that in the currently-received mutant base sequence information, and
a structure filter (305) that evaluates possibility of being a mutation causing the disease by increasing or decreasing the score depending on a determination result of determining whether a translocation, a reverse position, or a deletion has occurred in a vicinity of the positions of the enhancer stored in the storage unit (12),
the database filter (233) and the function prediction filter (234) increase or decrease the score by a value indicating the possibility of the mutation state being pathologic is high in the case where the database filter (233) determines that the mutation state is registered in the external apparatus as the mutation with pathogenicity and in the case where the database filter (233) determines that the mutation state is not registered in the external apparatus as a mutation state with pathogenicity and where the function prediction filter (234) evaluates that pathogenicity is present in the mutation state, as compared to the case where the database filter (233) determines that the mutation state is not registered in the external apparatus as a mutation state with pathogenicity and where the function prediction filter (234) evaluates that pathogenicity is not present in the mutation state.

2. The analysis apparatus according to claim 1, wherein the filter processing unit (23) further generates record information representing whether or not each of a plurality of the mutation states is the structural mutation, and
the output unit (25) outputs the record information in association with the rank information.

3. The analysis apparatus according to claim 1 or 2, wherein the filter processing unit determines the score depending on whether or not a fusion gene, in which a gene having a base sequence similar to a base sequence of a candidate gene is fused with another gene, is included in a base sequence corresponding to the mutation state, the candidate gene serving as a driver mutation candidate in a fusion gene fused with another gene.

4. The analysis apparatus according to claim 3, wherein the filter processing unit (23) determines the score depending on whether or not a fusion gene, in which two genes respectively similar to two candidate genes are fused, is included in the base sequence corresponding to the mutation state, the two candidate genes serving as driver mutation candidates in a fusion gene in which the candidate genes of a specific combination are fused.

5. The analysis apparatus according to any one of claims 1 to 4, wherein the filter processing unit determines the score depending on whether or not a position of a conserved sequence, the conserved sequence being a base sequence conserved between genomes of different organism species, is included in the mutation location of the mutation state.

6. The analysis apparatus according to any one of claims 1 to 5, wherein the filter processing unit determines the score depending on whether or not the mutation state is a chromosome translocation.

7. The analysis apparatus according to any one of claims 1 to 6, wherein the filter processing unit determines the score depending on whether or not the mutation state is a deletion of a registered gene that is registered in advance.

8. An analysis method for analyzing a mutation state, including a mutation location in a base sequence and the content of the mutation, extracted from genetic information of a sample to be analyzed by sequence alignment, the analysis method comprising the computer-implemented steps of:
receiving mutant base sequence information representing the mutation state;
retaining the received mutant base sequence information;
evaluating possibility of being a mutation causing a disease by
determining a score depending on a prediction result of predicting whether or not the mutation state affects protein function if it is determined that the mutation state is not registered in an external apparatus as a mutation with pathogenicity after (i) determining whether or not a mutation state represented by the received mutant base sequence information is a structural mutation across multiple genes and (ii) determining whether or not the mutation state is registered in the external apparatus as a mutation with pathogenicity,
increasing or decreasing the score depending on a determination result of determining whether or not the mutant base sequence information that is extracted from genetic information of an individual common to the mutant base sequence information currently received and that is previously received represents the same mutation state as that in the currently-received mutant base sequence information, and
increasing or decreasing the score depending on a determination result of determining, by referencing a storage unit that stores positions of an enhancer that controls expression of genes involved in cell canceration, whether a translocation, a reverse position, or a deletion has occurred in a vicinity of the positions of the enhancer stored in the storage unit;
outputting the determined score; and
creating rank information representing the degree of possibility that the mutation state is pathologic on the basis of the output score and outputting the created rank information, wherein
the evaluating possibility of being the mutation causing the disease includes determining, as the score, a value indicating that the possibility of the mutation state being pathologic is high in the case where it is determined that the mutation state is registered in the external apparatus as a mutation with pathogenicity and in the case where it is determined that the mutation state is not registered in the external apparatus as a mutation state with pathogenicity and where it is evaluated that pathogenicity is present in the mutation state, as compared to the case where it is determined that the mutation state is not registered in the external apparatus as a mutation state with pathogenicity and where it is evaluated that pathogenicity is not present in the mutation state.

9. A program that causes a computer to execute an analysis method for analyzing a mutation state, including a mutation location in a base sequence and the content of the mutation, extracted from genetic information of a sample to be analyzed by sequence alignment, the program causing the computer to execute the steps of:
receiving mutant base sequence information representing the mutation state;
retaining the received mutant base sequence information;
evaluating possibility of being a mutation causing a disease by
determining a score depending on a prediction result of predicting whether or not the mutation state affects protein function if it is determined that the mutation state is not registered in an external apparatus as a mutation with pathogenicity after (i) determining whether or not a mutation state represented by the received mutant base sequence information is a structural mutation across multiple genes and (ii) determining whether or not the mutation state is registered in the external apparatus as a mutation with pathogenicity,
increasing or decreasing the score depending on a determination result of determining whether or not the mutant base sequence information that is extracted from genetic information of an individual common to the mutant base sequence information currently received and that is previously received represents the same mutation state as that in the currently-received mutant base sequence information, and
increasing or decreasing the score depending on a determination result of determining, by referencing a storage unit that stores positions of an enhancer that controls expression of genes involved in cell canceration, whether a translocation, a reverse position, or a deletion has occurred in a vicinity of the positions of the enhancer stored in the storage unit;
outputting the determined score; and
creating rank information representing the degree of possibility that the mutation state is pathologic on the basis of the output score and outputting the created rank information, wherein
the evaluating possibility of being the mutation causing the disease includes determining, as the score, a value indicating that the possibility of the mutation state being pathologic is high in the case where it is determined that the mutation state is registered in the external apparatus as a mutation with pathogenicity and in the case where it is determined that the mutation state is not registered in the external apparatus as a mutation state with pathogenicity and where it is evaluated that pathogenicity is present in the mutation state, as compared to the case where it is determined that the mutation state is not registered in the external apparatus as a mutation state with pathogenicity and where it is evaluated that pathogenicity is not present in the mutation state.

## Patentansprüche

1. Analyseeinrichtung zum Analysieren eines Mutationszustands, der eine Mutationsstelle in einer Basensequenz und den Inhalt der Mutation enthält, die aus genetischen Informationen einer zu analysierenden Probe durch Sequenzalignment extrahiert wurden, umfassend:
eine Empfangseinheit (21), die mutierte Basensequenzinformationen empfängt, die den Mutationszustand darstellen;
eine Filterverarbeitungseinheit (23), die eine Bewertung ausgibt, die in Abhängigkeit davon bestimmt wird, ob der durch die von der Empfangseinheit (21) empfangenen mutierten Basensequenzinformationen dargestellte Mutationszustand eine strukturelle Mutation über mehrere Gene hinweg ist oder nicht;
eine Ausgabeeinheit (25), die Ranginformationen erstellt, die den Grad der Wahrscheinlichkeit darstellen, dass der Mutationszustand pathologisch ist, basierend auf der von der Filterverarbeitungseinheit (23) ausgegebenen Bewertung, und die erstellten Ranginformationen ausgibt;
eine Sicherungseinheit, die die von der Empfangseinheit (21) empfangenen mutierten Basensequenzinformationen sichert, und
eine Speichereinheit (12), die Positionen auf einem Chromosom eines Enhancers speichert, der eine Expression von Genen steuert, die an einer Zellkrebsentstehung beteiligt sind, wobei
die Filterverarbeitungseinheit (23) weiter enthält
einen Datenbankfilter (233), der bestimmt, ob der Mutationszustand in einer externen Einrichtung als eine Mutation mit Pathogenität registriert ist oder nicht,
einen Funktionsvorhersagefilter (234), der die Wahrscheinlichkeit, dass es sich um eine krankheitsverursachende Mutation handelt, bewertet, indem er die Bewertung in Abhängigkeit von einem Vorhersageergebnis erhöht oder verringert, das vorhersagt, ob der Mutationszustand die Proteinfunktion beeinflusst, wenn der Datenbankfilter (233) bestimmt, dass der Mutationszustand nicht in der externen Einrichtung als die Mutation mit Pathogenität registriert ist,
einen Zeitreihenfilter (232), der die Wahrscheinlichkeit, dass es sich um eine krankheitsverursachende Mutation handelt, durch Erhöhen oder Verringern der Bewertung in Abhängigkeit von einem Bestimmungsergebnis bewertet, bei dem bestimmt wird, ob die Mutationsbasensequenzinformationen, die aus den genetischen Informationen einer Person extrahiert werden, die den Mutationsbasensequenzinformationen gemeinsam sind, die derzeit von der Empfangseinheit (21) empfangen werden und die zuvor von der Empfangseinheit (21) empfangen wurden, denselben Mutationszustand wie in den derzeit empfangenen Mutationsbasensequenzinformationen darstellen, und
einen Strukturfilter (305), der die Wahrscheinlichkeit, dass es sich um eine krankheitsverursachende Mutation handelt, durch Erhöhen oder Verringern der Bewertung in Abhängigkeit von einem Bestimmungsergebnis bewertet, bei dem bestimmt wird, ob eine Translokation, eine umgekehrte Position oder eine Deletion in einer Nähe der Positionen des Enhancers, die in der Speichereinheit (12) gespeichert sind, aufgetreten ist,
wobei der Datenbankfilter (233) und der Funktionsvorhersagefilter (234) die Bewertung um einen Wert erhöhen oder verringern, der angibt, dass die Wahrscheinlichkeit hoch ist, dass der Mutationszustand pathologisch ist, in dem Fall, in dem der Datenbankfilter (233) bestimmt, dass der Mutationszustand in der externen Einrichtung als die Mutation mit Pathogenität registriert ist, und in dem Fall, in dem der Datenbankfilter (233) bestimmt, dass der Mutationszustand in der externen Einrichtung nicht als ein Mutationszustand mit Pathogenität registriert ist, und in dem der Funktionsvorhersagefilter (234) bewertet, dass Pathogenität in dem Mutationszustand vorhanden ist, im Vergleich zu dem Fall, in dem der Datenbankfilter (233) bestimmt, dass der Mutationszustand nicht in der externen Einrichtung als ein Mutationszustand mit Pathogenität registriert ist, und in dem der Funktionsvorhersagefilter (234) bewertet, dass der Pathogenität in dem Mutationszustand nicht vorhanden ist.

2. Analyseeinrichtung nach Anspruch 1, wobei die Filterverarbeitungseinheit (23) weiter Aufzeichnungsinformationen erzeugt, die darstellen, ob jeder einer Vielzahl von Mutationszuständen die strukturelle Mutation ist oder nicht, und
die Ausgabeeinheit (25) die Aufzeichnungsinformationen in Verbindung mit den Ranginformationen ausgibt.

3. Analyseeinrichtung nach Anspruch 1 oder 2, wobei die Filterverarbeitungseinheit die Bewertung in Abhängigkeit davon bestimmt, ob ein Fusionsgen, in dem ein Gen, das eine Basensequenz aufweist, die einer Basensequenz eines Kandidatengens ähnlich ist, mit einem anderen Gen fusioniert ist, in einer Basensequenz enthalten ist, die dem Mutationszustand entspricht, wobei das Kandidatengen als ein Treibermutationskandidat in einem mit einem anderen Gen fusionierten Fusionsgen dient.

4. Analyseeinrichtung nach Anspruch 3, wobei die Filterverarbeitungseinheit (23) die Bewertung in Abhängigkeit davon bestimmt, ob ein Fusionsgen, in dem zwei Gene, die jeweils zwei Kandidatengenen ähnlich sind, fusioniert sind, in der Basensequenz enthalten ist, die dem Mutationszustand entspricht, wobei die beiden Kandidatengene als Treibermutationskandidaten in einem Fusionsgen dienen, in dem die Kandidatengene einer bestimmten Kombination fusioniert sind.

5. Analyseeinrichtung nach einem der Ansprüche 1 bis 4, wobei die Filterverarbeitungseinheit die Bewertung in Abhängigkeit davon bestimmt, ob eine Position einer konservierten Sequenz, wobei die konservierte Sequenz eine zwischen Genomen verschiedener Organismusarten konservierte Basensequenz ist, in der Mutationsstelle des Mutationszustands enthalten ist.

6. Analyseeinrichtung nach einem der Ansprüche 1 bis 5, wobei die Filterverarbeitungseinheit die Bewertung in Abhängigkeit davon bestimmt, ob der Mutationszustand eine Chromosomentranslokation ist oder nicht.

7. Analyseeinrichtung nach einem der Ansprüche 1 bis 6, wobei die Filterverarbeitungseinheit die Bewertung in Abhängigkeit davon bestimmt, ob der Mutationszustand eine Deletion eines registrierten Gens, das zuvor registriert wurde, ist oder nicht.

8. Analyseverfahren zum Analysieren eines Mutationszustands, der eine Mutationsstelle in einer Basensequenz und den Inhalt der Mutation enthält, die aus genetischen Informationen einer zu analysierenden Probe durch Sequenzalignment extrahiert wurden, wobei das Analyseverfahren die computerimplementierten Schritte umfasst von:
Empfangen von mutierten Basensequenzinformationen, die den Mutationszustand darstellen;
Sichern der empfangenen mutierten Basensequenzinformationen;
Bewerten einer Wahrscheinlichkeit, dass es sich um eine krankheitsverursachende Mutation handelt, durch
Bestimmen einer Bewertung in Abhängigkeit von einem Vorhersageergebnis, das vorhersagt, ob der Mutationszustand eine Proteinfunktion beeinflusst oder nicht, wenn bestimmt wird, dass der Mutationszustand nicht in einer externen Einrichtung als eine Mutation mit Pathogenität registriert ist, nachdem (i) bestimmt wurde, ob ein durch die empfangenen mutierten Basensequenzinformationen dargestellter Mutationszustand eine strukturelle Mutation über mehrere Gene hinweg ist, und (ii) bestimmt wurde, ob der Mutationszustand in der externen Einrichtung als eine Mutation mit Pathogenität registriert ist,
Erhöhen oder Verringern der Bewertung in Abhängigkeit von einem Bestimmungsergebnis, bei dem bestimmt wird, ob die mutierten Basensequenzinformationen, die aus den genetischen Informationen einer Person extrahiert wurden, die mit den derzeit empfangenen mutierten Basensequenzinformationen gemeinsam sind und die zuvor empfangen wurden, denselben Mutationszustand wie die derzeit empfangenen mutierten Basensequenzinformationen darstellen, und
Erhöhen oder Verringern der Bewertung in Abhängigkeit von einem Bestimmungsergebnis, bei dem durch Referenzieren einer Speichereinheit, die Positionen eines Enhancers speichert, der eine Expression von Genen steuert, die an einer Zellkrebsentstehung beteiligt sind, bestimmt wird, ob eine Translokation, eine umgekehrte Position oder eine Deletion in einer Nähe der Positionen des in der Speichereinheit gespeicherten Enhancers aufgetreten ist;
Ausgeben der bestimmten Bewertung; und
Erstellen von Ranginformationen, die den Grad der Wahrscheinlichkeit darstellen, dass der Mutationszustand pathologisch ist, auf der Grundlage der ausgegebenen Bewertung, und Ausgeben der erstellten Ranginformationen, wobei das Bewerten der Wahrscheinlichkeit, dass die Mutation die Krankheit verursacht, das Bestimmen eines Wertes als die Bewertung enthält, der angibt, dass die Wahrscheinlichkeit, dass der Mutationszustand pathologisch ist, hoch ist, in dem Fall, in dem bestimmt wird, dass der Mutationszustand in der externen Einrichtung als eine Mutation mit Pathogenität registriert ist, und in dem Fall, in dem bestimmt wird, dass der Mutationszustand in der externen Einrichtung nicht als ein Mutationszustand mit Pathogenität registriert ist und in dem bewertet wird, dass Pathogenität in dem Mutationszustand vorliegt, im Vergleich zu dem Fall, in dem bestimmt wird, dass der Mutationszustand nicht in der externen Einrichtung als ein Mutationszustand mit Pathogenität registriert ist, und in dem bewertet wird, dass in dem Mutationszustand keine Pathogenität vorliegt.

9. Programm, das einen Computer veranlasst, ein Analyseverfahren zum Analysieren eines Mutationszustands auszuführen, der eine Mutationsstelle in einer Basensequenz und den Inhalt der Mutation enthält, die aus genetischen Informationen einer zu analysierenden Probe durch Sequenzalignment extrahiert wurden, wobei das Programm den Computer veranlasst, die Schritte auszuführen von:
Empfangen von mutierten Basensequenzinformationen, die den Mutationszustand darstellen;
Sichern der empfangenen mutierten Basensequenzinformationen;
Bewerten einer Wahrscheinlichkeit, dass es sich um eine krankheitsverursachende Mutation handelt, durch
Bestimmen einer Bewertung in Abhängigkeit von einem Vorhersageergebnis, bei dem vorhergesagt wird, ob der Mutationszustand eine Proteinfunktion beeinflusst oder nicht, wenn bestimmt wird, dass der Mutationszustand nicht in einer externen Einrichtung als eine Mutation mit Pathogenität registriert ist, nachdem (i) bestimmt wurde, ob ein durch die empfangenen mutierten Basensequenzinformationen dargestellter Mutationszustand eine strukturelle Mutation über mehrere Gene hinweg ist oder nicht, und (ii) bestimmt wurde, ob der Mutationszustand in der externen Einrichtung als eine Mutation mit Pathogenität registriert ist oder nicht,
Erhöhen oder Verringern der Bewertung in Abhängigkeit von einem Bestimmungsergebnis, bei dem bestimmt wird, ob die mutierten Basensequenzinformationen, die aus den genetischen Informationen einer Person extrahiert wurden, die mit den derzeit empfangenen mutierten Basensequenzinformationen gemeinsam sind und die zuvor empfangen wurden, denselben Mutationszustand wie die derzeit empfangenen mutierten Basensequenzinformationen darstellen, und
Erhöhen oder Verringern der Bewertung in Abhängigkeit von einem Bestimmungsergebnis, bei dem durch Referenzieren auf eine Speichereinheit, die Positionen eines Enhancers speichert, der eine Expression von Genen steuert, die an einer Zellkrebsentstehung beteiligt sind, bestimmt wird, ob in einer Nähe der in der Speichereinheit gespeicherten Positionen des Enhancers eine Translokation, eine umgekehrte Position oder eine Deletion aufgetreten ist;
Ausgeben der bestimmten Bewertung; und
Erstellen von Ranginformationen, die den Grad der Wahrscheinlichkeit darstellen, dass der Mutationszustand pathologisch ist, auf der Grundlage der ausgegebenen Bewertung, und Ausgeben der erstellten Ranginformationen, wobei das Bewerten der Wahrscheinlichkeit, dass die Mutation die Krankheit verursacht, das Bestimmen eines Wertes als die Bewertung enthält, der angibt, dass die Wahrscheinlichkeit, dass der Mutationszustand pathologisch ist, hoch ist, in dem Fall, in dem bestimmt wird, dass der Mutationszustand in der externen Einrichtung als eine Mutation mit Pathogenität registriert ist, und in dem Fall, in dem bestimmt wird, dass der Mutationszustand in der externen Einrichtung nicht als ein Mutationszustand mit Pathogenität registriert ist und in dem bewertet wird, dass Pathogenität in dem Mutationszustand vorhanden ist, im Vergleich zu dem Fall, in dem bestimmt wird, dass der Mutationszustand nicht in der externen Einrichtung als ein Mutationszustand mit Pathogenität registriert ist, und in dem bewertet wird, dass keine Pathogenität in dem Mutationszustand vorliegt.

## Revendications

1. Appareil d'analyse pour analyser un état de mutation, y compris un emplacement de mutation dans une séquence de bases et le contenu de la mutation, extrait d'informations génétiques d'un échantillon à analyser par alignement de séquences, comprenant :
une unité de réception (21) qui reçoit des informations de séquence de bases mutante représentant l'état de mutation ;
une unité de traitement par filtres (23) qui fournit un score déterminé en fonction du fait que l'état de mutation représenté par les informations de séquence de bases mutante reçues par l'unité de réception (21) est ou non une mutation structurelle sur de multiples gènes ;
une unité de sortie (25) qui crée des informations de classement représentant le degré de possibilité que l'état de mutation soit pathologique sur la base du score fourni par l'unité de traitement par filtres (23) et fournit les informations de classement créées ;
une unité de conservation qui conserve les informations de séquence de bases mutante reçues par l'unité de réception (21), et
une unité de mémorisation (12) qui mémorise des positions sur un chromosome d'un amplificateur qui régule l'expression de gènes impliqués dans la cancérisation cellulaire, dans lequel
l'unité de traitement par filtres (23) comprend en outre
un filtre de base de données (233) qui détermine si l'état de mutation est ou non enregistré dans un appareil externe en tant que mutation à pathogénicité,
un filtre de prédiction de fonction (234) qui évalue la possibilité que la mutation soit une mutation causant une maladie en augmentant ou diminuant le score en fonction d'un résultat de prédiction consistant à prédire si l'état de mutation affecte ou non une fonction de protéine si le filtre de base de données (233) détermine que l'état de mutation n'est pas enregistré dans l'appareil externe en tant que mutation à pathogénicité,
un filtre de série temporelle (232) qui évalue la possibilité que la mutation soit une mutation causant la maladie en augmentant ou en diminuant le score en fonction d'un résultat de détermination consistant à déterminer si les informations de séquence de bases mutante qui sont extraites d'informations génétiques d'un individu commun aux informations de séquence de bases mutante actuellement reçues par l'unité de réception (21) et qui ont été précédemment reçues par l'unité de réception (21) représentent ou non le même état de mutation que celui dans les informations de séquence de bases mutante actuellement reçues, et
un filtre de structure (305) qui évalue la possibilité que la mutation soit une mutation causant la maladie en augmentant ou diminuant le score en fonction d'un résultat de détermination consistant à déterminer si une translocation, une position inverse ou une délétion a eu lieu dans un voisinage des positions de l'amplificateur mémorisées dans l'unité de mémorisation (12),
le filtre de base de données (233) et le filtre de prédiction de fonction (234) augmentent ou diminuent le score d'une valeur indiquant que la possibilité que l'état de mutation soit pathologique est élevée dans le cas où le filtre de base de données (233) détermine que l'état de mutation est enregistré dans l'appareil externe en tant que la mutation à pathogénicité et dans le cas où le filtre de base de données (233) détermine que l'état de mutation n'est pas enregistré dans l'appareil externe en tant qu'état de mutation à pathogénicité et où le filtre de prédiction de fonction (234) évalue que la pathogénicité est présente dans l'état de mutation, en comparaison du cas où le filtre de base de données (233) détermine que l'état de mutation n'est pas enregistré dans l'appareil externe en tant qu'état de mutation à pathogénicité et où le filtre de prédiction de fonction (234) évalue que la pathogénicité n'est pas présente dans l'état de mutation.

2. Appareil d'analyse selon la revendication 1, dans lequel l'unité de traitement par filtres (23) génère en outre des informations d'enregistrement indiquant si chacun parmi une pluralité des états de mutation est ou non la mutation structurelle, et
l'unité de sortie (25) fournit les informations d'enregistrement en association avec les informations de classement.

3. Appareil d'analyse selon la revendication 1 ou 2, dans lequel l'unité de traitement par filtres détermine le score en fonction du fait qu'un gène de fusion, dans lequel un gène ayant une séquence de bases analogue à une séquence de bases d'un gène candidat est fusionné avec un autre gène, est ou non inclus dans une séquence de bases correspondant à l'état de mutation, le gène candidat servant de candidat à une mutation conductrice dans un gène de fusion fusionné avec un autre gène.

4. Appareil d'analyse selon la revendication 3, dans lequel l'unité de traitement par filtres (23) détermine le score en fonction du fait qu'un gène de fusion, dans lequel sont fusionnés deux gènes respectivement analogues à deux gènes candidats, est ou non inclus dans la séquence de bases correspondant à l'état de mutation, les deux gènes candidats servant de candidats à une mutation conductrice dans un gène de fusion dans lequel sont fusionnés les gènes candidats d'une combinaison spécifique.

5. Appareil d'analyse selon l'une quelconque des revendications 1 à 4, dans lequel l'unité de traitement par filtres détermine le score en fonction du fait qu'une position d'une séquence conservée, la séquence conservée étant une séquence de bases conservée entre des génomes de différentes espèces d'organismes, est ou non incluse dans l'emplacement de mutation de l'état de mutation.

6. Appareil d'analyse selon l'une quelconque des revendications 1 à 5, dans lequel l'unité de traitement par filtres détermine le score en fonction du fait que l'état de mutation est ou non une translocation chromosomique.

7. Appareil d'analyse selon l'une quelconque des revendications 1 à 6, dans lequel l'unité de traitement par filtres détermine le score en fonction du fait que l'état de mutation est ou non une délétion d'un gène enregistré qui est enregistré à l'avance.

8. Procédé d'analyse pour analyser un état de mutation, y compris un emplacement de mutation dans une séquence de bases et le contenu de la mutation, extrait d'informations génétiques d'un échantillon à analyser par alignement de séquences, le procédé d'analyse comprenant les étapes implémentées sur ordinateur, consistant à :
recevoir des informations de séquence de bases mutante représentant l'état de mutation ;
conserver les informations de séquence de base mutante reçues ;
évaluer la possibilité que la mutation soit une mutation causant une maladie en
déterminant un score en fonction d'un résultat de prédiction consistant à prédire si l'état de mutation affecte ou non une fonction de protéine s'il est déterminé que l'état de mutation n'est pas enregistré dans un appareil externe en tant que mutation à pathogénicité après (i) avoir déterminé si un état de mutation représenté par les informations de séquence de bases mutante reçues est ou non une mutation structurelle sur de multiples gènes et (ii) avoir déterminé si l'état de mutation est ou non enregistré dans l'appareil externe en tant que mutation à pathogénicité,
augmenter ou diminuer le score en fonction d'un résultat de détermination consistant à déterminer si les informations de séquence de bases mutante qui sont extraites d'informations génétiques d'un individu commun aux informations de séquence de bases mutante actuellement reçues et qui ont été précédemment reçues représentent ou non le même état de mutation que celui dans les informations de séquence de bases mutante actuellement reçues, et
augmenter ou diminuer le score en fonction d'un résultat de détermination consistant à déterminer, en se rapportant à une unité de mémorisation qui mémorise les positions d'un amplificateur qui régule l'expression de gènes impliqués dans la cancérisation cellulaire, si une translocation, une position inverse ou une délétion a eu lieu dans un voisinage des positions de l'amplificateur mémorisées dans l'unité de mémorisation ;
fournir le score déterminé ; et
créer des informations de classement représentant le degré de possibilité que l'état de mutation soit pathologique sur la base du score fourni et fournir les informations de classement créées, dans lequel
l'évaluation de la possibilité que la mutation soit une mutation causant la maladie comprend déterminer, en tant que le score, une valeur indiquant que la possibilité que l'état de mutation soit pathologique est élevée dans le cas où il est déterminé que l'état de mutation est enregistré dans l'appareil externe en tant que mutation à pathogénicité et dans le cas où il est déterminé que l'état de mutation n'est pas enregistré dans l'appareil externe en tant qu'état de mutation à pathogénicité et où il est évalué que la pathogénicité est présente dans l'état de mutation, en comparaison du cas où il est déterminé que l'état de mutation n'est pas enregistré dans l'appareil externe en tant qu'état de mutation à pathogénicité et où il est évalué que la pathogénicité n'est pas présente dans l'état de mutation.

9. Programme qui amène un ordinateur à exécuter un procédé d'analyse pour analyser un état de mutation, y compris un emplacement de mutation dans une séquence de bases et le contenu de la mutation, extrait d'informations génétiques d'un échantillon à analyser par alignement de séquences, le programme amenant l'ordinateur à exécuter les étapes consistant à :
recevoir des informations de séquence de bases mutante représentant l'état de mutation ;
conserver les informations de séquence de base mutante reçues ;
évaluer la possibilité que la mutation soit une mutation causant une maladie en
déterminant un score en fonction d'un résultat de prédiction consistant à prédire si l'état de mutation affecte ou non une fonction de protéine s'il est déterminé que l'état de mutation n'est pas enregistré dans un appareil externe en tant que mutation à pathogénicité après (i) avoir déterminé si un état de mutation représenté par les informations de séquence de bases mutante reçues est ou non une mutation structurelle sur de multiples gènes et (ii) avoir déterminé si l'état de mutation est ou non enregistré dans l'appareil externe en tant que mutation à pathogénicité,
augmenter ou diminuer le score en fonction d'un résultat de détermination consistant à déterminer si les informations de séquence de bases mutante qui sont extraites d'informations génétiques d'un individu commun aux informations de séquence de bases mutante actuellement reçues et qui ont été précédemment reçues représentent ou non le même état de mutation que celui dans les informations de séquence de bases mutante actuellement reçues, et
augmenter ou diminuer le score en fonction d'un résultat de détermination consistant à déterminer, en se rapportant à une unité de mémorisation qui mémorise les positions d'un amplificateur qui régule l'expression de gènes impliqués dans la cancérisation cellulaire, si une translocation, une position inverse ou une délétion a eu lieu dans un voisinage des positions de l'amplificateur mémorisées dans l'unité de mémorisation ;
fournir le score déterminé ; et
créer des informations de classement représentant le degré de possibilité que l'état de mutation soit pathologique sur la base du score fourni et fournir les informations de classement créées, dans lequel
l'évaluation de la possibilité que la mutation soit une mutation causant la maladie comprend déterminer, en tant que le score, une valeur indiquant que la possibilité que l'état de mutation soit pathologique est élevée dans le cas où il est déterminé que l'état de mutation est enregistré dans l'appareil externe en tant que mutation à pathogénicité et dans le cas où il est déterminé que l'état de mutation n'est pas enregistré dans l'appareil externe en tant qu'état de mutation à pathogénicité et où il est évalué que la pathogénicité est présente dans l'état de mutation, en comparaison du cas où il est déterminé que l'état de mutation n'est pas enregistré dans l'appareil externe en tant qu'état de mutation à pathogénicité et où il est évalué que la pathogénicité n'est pas présente dans l'état de mutation.
